# FASCICULE DE BREVET EUROPEEN

(11) **EP 3 673 048 B1**
(45) Date de publication et mention de la délivrance du brevet: **27.11.2024**
(21) Numéro de dépôt: 18758650.8
(22) Date de dépôt: 11.06.2018
(51) Int. Cl.: C12N 5/077, A61K 35/34, A61L 27/38, A61L 27/52, A61L 27/54, A61L 27/56

(54) **MATÉRIEL ET MÉTHODE POUR LE STOCKAGE, LE TRANSFERT ET LA DÉLIVRANCE DE CELLULES SOUCHES MESENCHYMATEUSES IMMÉDIATEMENT DISPONIBLES ET FONCTIONNELLES DANS UN CONTEXTE D'INFARCTUS DU MYOCARDE**
MATERIAL UND VERFAHREN ZUR LAGERUNG, ÜBERTRAGUNG UND ABGABE VON MESENCHYMALEN STAMMZELLEN, DIE IM ZUSAMMENHANG MIT EINEM HERZINFARKT SOFORT VERFÜGBAR UND FUNKTIONSFÄHIG SIND
MATERIAL AND METHOD FOR STORING, TRANSFERRING AND DELIVERING MESENCHYMAL STEM CELLS WHICH ARE IMMEDIATELY AVAILABLE AND FUNCTIONAL IN THE CONTEXT OF A MYOCARDIAL INFARCTION

(30) Priorité: 25.08.2017 FR 1757884
(43) Date de publication de la demande: 01.07.2020
(73) Titulaire: SCHUSSLER, Olivier, 74140 Messery (FR)
(72) Inventeur: SCHUSSLER, Olivier, 74140 Messery (FR)
(74) Mandataire: Grosset-Fournier, Chantal Catherine
(86) Numéro de dépôt international: PCT/FR2018/000168
(87) Numéro de publication internationale: WO 2019/038484

(56) Documents cités:
- FERNANDEZ-REBOLLO EDUARDO ET AL: "Human Platelet Lysate versus Fetal Calf Serum: These Supplements Do Not Select for Different Mesenchymal Stromal Cells", SCIENTIFIC REPORTS, vol. 7, no. 1, 11 July 2017 (2017-07-11), XP055843294, Retrieved from the Internet <URL:https://www.nature.com/articles/s41598-017-05207-1.pdf> DOI: 10.1038/s41598-017-05207-1
- YUN-LI LIN ET AL: "Stiffness-controlled three-dimensional collagen scaffolds for differentiation of human Wharton's jelly mesenchymal stem cells into cardiac progenitor cells : Collagen Scaffold for MSC Differentiate into Cardiomyogenic Cells", JOURNAL OF BIOMEDICAL MATERIALS RESEARCH. PART A, vol. 104, no. 9, 6 May 2016 (2016-05-06), HOBOKEN, NY, US, pages 2234 - 2242, XP055460309, ISSN: 1549-3296, DOI: 10.1002/jbm.a.35762
- JENNIFER S PARK ET AL: "The effect of matrix stiffness on the differentiation of mesenchymal stem cells in response to TGF-", BIOMATERIALS, ELSEVIER SCIENCE PUBLISHERS BV., BARKING, GB, vol. 32, no. 16, 10 February 2011 (2011-02-10), pages 3921 - 3930, XP028370855, ISSN: 0142-9612, [retrieved on 20110217], DOI: 10.1016/J.BIOMATERIALS.2011.02.019
- FLOREN MICHAEL ET AL: "Human mesenchymal stem cells cultured on silk hydrogels with variable stiffness and growth factor differentiate into mature smooth muscle cell phenotype", ACTA BIOMATERIALIA, ELSEVIER, AMSTERDAM, NL, vol. 31, 24 November 2015 (2015-11-24), pages 156 - 166, XP029392550, ISSN: 1742-7061, DOI: 10.1016/J.ACTBIO.2015.11.051
- JENNIFER DAWSON ET AL: "Collagen scaffolds with or without the addition of RGD peptides support cardiomyogenesis after aggregation of mouse embryonic stem cells", IN VITRO CELLULAR & DEVELOPMENTAL BIOLOGY - ANIMAL, SPRINGER-VERLAG, NEW YORK, vol. 47, no. 9, 23 September 2011 (2011-09-23), pages 653 - 664, XP019973211, ISSN: 1543-706X, DOI: 10.1007/S11626-011-9453-0

## Description

### DOMAINE TECHNIQUE

La demande décrit une méthode qui comprend le fait de différentier des cellules souches mésenchymateuses (MSC) en cellules contractiles et d'associer les cellules différenciées à un support 3D afin d'obtenir un tissu contractile. L'activité paracrine des MSC est induite au cours du processus. Les biomatériaux obtenus sont compatibles avec le stockage, le transfert, et la délivrance de MSC à basse température. Les préparations sont immédiatement disponibles et fonctionnelles.

La demande décrit donc un biomatériau cellulaire contractile, un procédé de production de ce biomatériau ainsi que des applications médicales de ce biomatériau. Le biomatériau de la demande est particulièrement adapté à la préservation, l'angiogenèse, le contrôle du remodelage et la régénération de tissu myocardique notamment tissus myocardique infarci.

### ARRIÈRE-PLAN TECHNOLOGIQUE

Le tissu myocardique peut subir des lésions qui diminuent sa contractilité. Ces lésions myocardiques peuvent conduire le patient à une insuffisance cardiaque. Elles peuvent avoir différentes origines, par exemple une origine virale, une origine génétique ou une origine ischémique. La principale cause de lésions myocardiques est l'infarctus du myocarde. L'infarctus du myocarde surgit généralement du fait de l'obstruction d'une ou plusieurs artères coronaires, qui prive de l'apport en sang, et donc en oxygène, dont les cellules du tissu myocardique ont besoin, conduisant ainsi à une nécrose d'une ou plusieurs zones du myocarde. L'infarctus du myocarde est une pathologie extrêmement fréquente dans les pays industrialisés (1/500), survenant plutôt chez des sujets âgés de plus de 50 ans, avec 25% de formes graves.

Même lorsqu'il est traité à temps dans les 2h, par exemple par réouverture des artères obstruées (angioplastie), l'infarctus du myocarde conduit généralement à une insuffisance cardiaque avec perte plus ou moins importante de contractilité myocardique. L'insuffisance cardiaque secondaire a une mortalité à 5 ans du même ordre que les cancers avec 50% de mortalité.

La diminution de la contractilité myocardique est notamment due à la perte (par nécrose) de cellules musculaires cardiaques. Elle est également due à une perte de matrice extracellulaire myocardique ainsi qu'à une perte en cellules de soutien de cette matrice extracellulaire, notamment à une perte en fibroblastes cardiaques. Or, cette matrice extracellulaire et ces cellules de soutien sont nécessaires à la régénération des cellules musculaires cardiaques, qui sont des cellules adhérentes. La perte de matrice et de cellules de soutien limite donc les possibilités de régénération des cellules musculaires cardiaques. Elle conduit en outre à un remodelage du ventricule, qui doit s'adapter au nouveau régime de pression sanguine appliqué. La perte de cellules et de matrice extracellulaire s'accompagne en outre d'une réaction inflammatoire (initiée notamment par les débris de cellules et de matrice cellulaire). Lors de l'infarctus, des mécanismes sont mis en jeu très rapidement. La nécrose des cellules, fragments matrice extracellulaire et les cellules locales vont recruter des cellules inflammatoires initialement des polynucléaires neutrophiles (PNN) à 8h, puis des macrophages M1 après 24h. Sous l'effet des myofibroblastes locaux il va y avoir une transformation des macrophages de type M1 en macrophages de type M2 régénératifs, angiogéniques, peu détersifs. L'accélération de la présence des macrophages M2 améliore la récupération et la préservation du tissu myocardique. Les myofibroblastes sont de plus en plus reconnus comme les acteurs principaux de la régulation de la réponse après infarctus.

Le tissu myocardique post-infarctus est donc un tissu ischémique et inflammatoire, qui a subi une perte cellulaire et une perte de matrice extracellulaire.

Le corps humain est théoriquement capable d'une certaine récupération, régénération myocardique, contrôlée par les myofibroblastes naturellement générés par différenciation de fibroblastes locaux ou issus de la moelle osseuse. Néanmoins, cette transformation naturelle n'est pas immédiate : la transformation des fibroblastes en myofibroblastes met au moins une semaine et donc ces cellules ne sont pas présentent à la phase aigüe, et la réaction inflammatoire qui se développe en suite de l'infarctus tend à conduire à un remodelage du ventricule et à une altération de la contractilité ventriculaire.

Pour les formes les plus avancées d'insuffisance cardiaque, la transplantation cardiaque et/ou l'assistance mécanique restent un traitement possible. Le problème de ces assistances est que les meilleures survies ne sont que de quelques années, elles sont très chères, avec beaucoup d'accidents d'anticoagulations et des conditions de vie altérées. Les transplantations cardiaques sont réservées aux sujets jeune car il y a un manque cruel de greffons. Il s'agit d'intervention très lourde avec une survie de 50% à 10 ans et de nombreuses complications avec les traitements immunosuppresseurs et cancers secondaires, rejets des greffons *etc.* Pour l'infarctus qui est une maladie extrêmement fréquente et grave il faut donc trouver un traitement qui soit efficace, peu cher et pouvant être utilisé à tous les âges.

En cas d'infarctus avec choc cardiogénique initial, il n'y a pratiquement pas eu de progrès depuis 15 ans même avec des revascularisations précoces avec 50% de mortalité hospitalière et 50% de mortalité pour les survivants lors de la première année.

La thérapie cellulaire a suscité bien des espérances pour améliorer les résultats dans le cas de l'infarctus mais avec pour l'instant peu d'application clinique car les résultats sont trop décevants même si certains résultats chez l'animal sont prometteurs. Une des explications des différences entre les études cliniques et les études chez l'animal pourrait être la disponibilité supérieure des cellules pour des raisons de logistique dans un contexte d'infarctus. Il n'y a pas chez l'homme de préparation immédiatement disponible. Les thérapies sont effectuées avec délai et les cellules ne sont pas utilisées avec des fonctionnalités optimales.

Des thérapies cellulaires ont donc été envisagées à des fins de préservation, de cicatrisation ou de régénération du tissu myocardique. Ont été testés différents types cellulaires, notamment des cellules différenciées (par exemple des cardiomyocytes) et des cellules souches (par exemple des cellules souches mésenchymateuses). Tous les résultats avec les cellules MSC bien que ce soient des cellules prometteuses ne s'accompagnent que d'une amélioration de la fonction ventriculaire contractile très faible (moins de 5% seulement alors qu'il faudrait une amélioration supérieure à 5% pour avoir un gain en termes de survie chez les patients). Chez l'animal, il a été montré que des MSC allogénique administrées immédiatement après l'infarctus et modifiées avec des gènes de survie notamment AkT ou Bcl2 pouvaient entrainer une amélioration de la contractilité systolique. Il a été montré également une corrélation entre le niveau de prise de greffe initial des cellules et la récupération. Les cellules doivent être appliquées très précocement. Un des modes d'action rapporté des MSC *in vivo* est le switch entre les cellules de détersion initiales macrophages M1 vers des macrophages angiogéniques régénératifs de type M2. Si les MSC sont administrées trop tardivement, leur effet est diminué.

Les cellules différenciées, telles que des cardiomyocytes, posent le problème de la difficulté d'obtenir des cellules adultes en quantité suffisante. Par suite, les choix se sont davantage orientés vers les cellules souches telles que les cellules souches mésenchymateuses.

Dans les thérapies cellulaires de l'art antérieur, on constate le plus souvent qu'il a une survie insuffisante des cellules administrées, et que le niveau de prise en greffe de ces cellules sur la zone infarcie est très limité avec perte de 90% après seulement 1h (facteur encore aggravé si le myocarde se contracte), de sorte que les cellules administrées peuvent migrer vers des sites qui sont sans rapport avec la zone ou le tissu à traiter.

Des patches de collagène ont été utilisés pour limiter la migration des cellules souches, notamment des cellules souches mésenchymateuses. Certains de ces patches ont pu conduire à une limitation du remodelage du ventricule avec, par exemple, une limitation de la distension diastolique, mais ces patches ne conduisent pas à une amélioration de la contractilité myocardique, notamment de la contractilité ventriculaire systolique (*cf.* Simpson et al., 2007 Stem Cells 25(9) : 2350-2357 ; plus particulièrement le tableau 2 de cette publication).

Le problème de la survie, de la viabilité et de la prise en greffe des cellules administrées est d'autant plus délicat que la zone à traiter est une zone ischémique et inflammatoire, c'est-à-dire non favorable à la survie et à la viabilité cellulaire.

La logistique pour transférer des cellules nécessite d'avoir au minimum un délai de 5-7 jours pour l'acheminement et la délivrance. Transférer les cellules à température de 37°C nécessite d'avoir des incubateurs portables, du CO₂ et de l'oxygène, la capacité de cultiver ces cellules à l'arrivée en conditions cGMP et des centres accrédités. D'autre part, les MSC sont des cellules adhérentes qui vont soit s'agglutiner entre elles, soit adhérer aux parois du container de transfert. La délivrance de ces cellules est donc techniquement problématique. À 37°C, le métabolisme des cellules est élevé. Les MSC sont des cellules multipotentes qui vont se différencier de manière anarchique. Si les cellules sont préservées à 37°C, il n'y a pas possibilité de contrôler cette différenciation. Au niveau demande métabolique, du fait de ses propriétés contractile, les tissus contractiles par rapport aux tissus non contractile à température ambiante sont particulièrement demandeurs de nutriments et d'oxygène.

Les thérapies cellulaires de l'art antérieur mettent généralement en oeuvre des cellules qui sont conservées par congélation. Comme cela est cité dans une étude de Marquez-Curtis et al. (review Cryobiology. 2015 Oct;71(2):181-97), « le monde du tissu engineering, de la thérapie génique, de la médecine régénérative et de la transplantation reposent sur la capacité à préserver, stocker et transporter ces cellules ». Pour des raisons de logistique avec les MSC, il est indiqué que la question n'est pas de savoir s'il faut les congeler puisqu'il n'y a pas d'autre solution, mais comment faire pour préserver leur fonctionnalité après congélation. La congélation est donc considérée comme indispensable avec les techniques actuelles. Les cellules mésenchymateuses sont des cellules en faible nombre dans les tissus et qui doivent donc être amplifiées *in vitro* en culture 2D. Lors des phases d'expansion, les cellules sont repiquées en utilisant des solutions enzymatiques. Lorsqu'un batch suffisant de MSC est amplifié, les MSC sont alors stockées. Un batch de test à visée de sécurités virales (possible mutation génétique) est effectué. La congélation permet également de maintenir les MSC dans un état de différenciation donné (pour stocker les MSC et les délivrer dans les centres). Après décongélation, en culture les cellules vont mettre plusieurs jours à récupérer leur fonctionnalité (au moins 72h). Ces MSC ne sont pas immédiatement disponibles dans un contexte d'infarctus et nécessitent une expertise et une accréditation du centre pouvant re-expandre ces cellules. Ces compétences bloquent l'utilisation de ces cellules.

Ces cellules doivent donc être décongelées avant d'être administrées au patient. L'étape de décongélation retarde d'autant la prise en charge thérapeutique du patient. En outre, quelle que soit la technique de cryopréservation mise en oeuvre, le fait de congeler et décongeler diminue la viabilité des cellules. Par exemple, le fait de congeler et décongeler des cellules souches mésenchymateuses s'accompagne d'une grande mortalité cellulaire, d'une anomalie ultra-structurale des cellules, d'une non-adhérence des cellules pendant plus de 24 heures et d'une perte de la capacité immunosuppressive paracrine de ces cellules pendant 48 à 72 heures après décongélation. Une perte de l'activité paracrine immuno-modulatrice a même pu être constatée à l'issue de la décongélation (Moll et al. 2014 Stem Cells 32(9) : 2430-2442). De manière générale, le fait de congeler et décongeler des cellules adhérentes, qu'il s'agisse de cellules souches mésenchymateuses ou de cellules contractiles, altère la capacité d'adhérence de ces cellules ou retarde le retour de leur capacité d'adhérence cellulaire. La récupération des cellules nécessite de cultiver les cellules en incubateur. Les cellules après décongélation n'étant pas adhérentes, si on les injecte avec un support, elles ne vont pas adhérer au support et la perte cellulaire initiale serait très importante. L'adhérence des cellules au support de contractilité et l'engagement des cellules dans des interactions de contractilité (avec augmentation et amélioration des interactions cellules/cellules et cellules/support) permet une meilleure rétention initiale des cellules dans la zone à traiter contrairement à l'injection de cellules en libre qui s'accompagne de la perte de 90% des cellules après 1h seulement et encore plus dans un coeur qui bat. L'approche proposée dans la demande permet d'optimiser les performances de la thérapie cellulaire par une meilleure rétention initiale des cellules et par une meilleure préservation du contingent cellulaire (meilleure rétention initiale, meilleure survie, moindre migration secondaire). Elle permet également d'appliquer des cellules au contact de, et non pas dans, la zone infarcie, ce qui permet de préserver le phénotype paracrine immunosuppressif pro-régénératif des cellules et de préserver leur état de différenciation.

Une approche pour délivrer les MSC pourrait être de cultiver les MSC en culture 2D en hypothermie pour ralentir leur métabolisme. Néanmoins, pour des températures basses inférieures à 10°C en milieu de culture classique avec sérum, 90% des MSC ou des cellules contractiles sont déjà mortes après seulement 24h (Mathew et al., Tissue Eng. 2004;10(11-12):1662-1671). À basse température >0°C il y a des modifications du cycle de production de l'ATP, dégradation de transporteurs membranaires (responsable de modifications des ions avec augmentation du calcium intracellulaire, perte du potassium intracellulaire et augmentation du sodium, acidose avec pH cytoplasmique à 4 normalement à 7). À basse température, la modification des propriétés viscoélastique des membranes conduit à des membranes plus rigides. Des cascades de dommage cellulaire sont mises en jeu avec la libération d'enzymes intracytoplasmiques, l'activation de phospholipase calcium dépendante, l'activation de voie de mort cellulaire par apoptose et la destruction de la matrice intracellulaire impliquée dans les phénomènes de contractilité (Baust et al., Organogenesis. 2009;5(3):90-96). Ce qui est remarquable, c'est que dans le milieu de culture classique avec sérum nous sommes capables de préserver les cellules pendant plus d'une semaine sans perte de cette contractilité.

Des solutions hypothermiques riches en lactobionate ont été proposées pour remplacer le chlore et diminuer sa concentration intracellulaire pour limiter la pression oncotique des solutions de type lactobionate sucrose et mannitol. Les solutions sont également tamponnées par Hepès.

Des solutions hypothermiques de type HYPERTHERMOSOL^{®} ont été testées avec des cellules progénitrices pour cardiomyocytes. Dans ces solutions, outre la survie diminuée avec le temps notamment après 4 jours et plus de 50% mortalité après 1 semaine (Van Burskirk et al., Bioprocess International. 2004:42-48), il existe une baisse très importante d'alpha-actinine après 48h (Herías, Cytotherapy, 16(4), S38). Il existe d'autre part une baisse très importante du marqueur CD9 (Ginis et al., Tissue Eng Part C Methods. 2012;18(6):453-463) qui est un marqueur des fibroblastes. Lors des phases de réchauffement, le milieu est toxique pour les cellules avec la libération de radicaux libres. Il faut donc changer le milieu lors du réchauffement, centrifuger les cellules et utiliser un incubateur pour régénérer les cellules. Après réchauffement, changement du milieu et culture en incubateur, il faut 24h à 48h pour que les cellules survivantes produisent des battements (Snyder et al., 2005, Cell Préservation Technology, 3(1), 61-74.).

Donc ces approches ne permettent pas d'avoir des préparations à plus de 5 jours alors qu'il faut un minimum de 7 jours pour des questions de logistiques pour transférer les préparations. Si on imagine que ces préparations vont servir pour un événement aléatoire il faut une capacité de durée de stockage supplémentaire ce que ne permet encore moins les approches précédentes.

Il en résulte qu'en pratique, en l'occurrence en clinique, les thérapies cellulaires destinées à la cicatrisation ou la régénération d'un tissu myocardique infarci présentent un faible rapport bénéfice / risque.

Le dispositif tel que décrit est immédiatement disponible. Il n'y a pas besoin de remonter la température à 37°C. À température ambiante le dispositif est contractile. Les cellules dans les supports de contractilité peuvent éventuellement être utilisées avec des solutions hypothermiques. Dans ce cas, comme les cellules sont déjà adhérentes au support, on peut laver les cellules et le support par simple changement de milieu. Dans le support de contractilité les interactions entre les cellules entre elles et entre les cellules et le support entrainent une activation du gène de survie AKt de manière physiologique et les protéines du stress sont diminuées. Cela pourrait expliquer la résistance des cellules dans cette structure en hypothermie.

Les moyens de thérapie régénérative (ou cicatricielle) du tissu myocardique, notamment du tissu myocardique infarci, tels que décrits présentent des avantages par rapport aux moyens de l'art antérieur, et notamment l'avantage de ne pas requérir la conservation sous forme congelée des cellules destinées à être administrées au patient ou sujet. Les moyens de la demande peuvent notamment conduire non seulement à limiter voire à éviter le remodelage du myocarde, mais également à une amélioration de la contractilité myocardique.

Par ailleurs, on connait également le document « Stiffness-controlled three-dimensional collagen scaffoids for différentiation of human Wharton's jelly mesenchymal stem cells into cardiac progenitor cells" (Yun-Li Lin et al., 2016) qui divulgue la fabrication de supports de collagène 3D contrôlés par la rigidité qui permettent la prolifération et la différenciation des cellules souches mésenchymateuses en cellules progénitrices cardiaques à la surface du support. On connait également le document *"*The effect of matrix stiffness on the différentiation of mesenchymal stem cells in response to TGF-β" (Park et al;, 2011) qui divulgue l'effet de la rigidité de la matrice sur la différenciation des cellules souches mésenchymateuses en réponse au TGFβ1, et à la surface du support. Enfin, on connait également le document *"*Human mesenchymal stem cells cultured on silk hydrogels with variable stiffness and growth factor differentiate into mature smooth muscle cell phenotype" (Floren et al;, 2016) qui divulgue l'effet de l'élasticité d'un hydrogel poreux de fibroïne sur la différenciation des cellules souches mésenchymateuses en réponse au TGFβ1. Cependant ces documents ne décrivent pas de biomatériau cellulaire contractile.

### RÉSUMÉ

L'invention est énoncée dans l'ensemble des revendications annexées.

En outre, la présente demande décrit un biomatériau, un procédé de production de ce biomatériau ainsi que des applications médicales de ce biomatériau.

Le biomatériau tel que décrit est contractile et sa contractilité est spontanée ou provoquée. En outre, il peut présenter une activité paracrine inductible (notamment une activité paracrine d'immunosuppression de cellules T).

Le biomatériau tel que décrit dans la demande est particulièrement adapté à la réparation (ou cicatrisation) du tissu myocardique, notamment du tissu myocardique infarci.

Le biomatériau tel que décrit dans la demande peut notamment favoriser la régénération myocardique et/ou favoriser l'angiogenèse au niveau de la zone infarcie. Le biomatériau de la demande peut diminuer la taille de la zone infarcie et/ou limiter voire éviter le remodelage du ventricule.

De manière particulièrement notable, le biomatériau tel que décrit dans la demande peut conduire à une moindre diminution ou amélioration de la contractilité myocardique.

Le biomatériau tel que décrit dans la demande ne nécessite pas d'être congelé pour être conservé : il peut être conservé à une température supérieure à 0°C sans pour autant perdre ses propriétés de contractilité (et éventuellement d'activité paracrine).

Le biomatériau tel que décrit dans la demande comprend un support tridimensionnel à complaisance d'élasticité, qui favorise la survie et la différenciation de cellules souches en cellules contractiles et qui permet la formation d'un tissu contractile. Le support peut être un support solide, et la colonisation cellulaire du support peut être favorisé par une structure poreuse.

Le tissu contractile est formé par la différenciation de cellules souches, notamment de cellules souches mésenchymateuses. Les cellules souches mésenchymateuses de la demande incluent également des préparations contenant des MSC comme des « clusters » de type cardiosphère préparés à partir de fragments tissulaires cardiaques et enrichis en progéniteurs pour CKIT+ et MSC.

Le tissu contractile peut, par exemple, comprendre ou être constitué de cellules choisies parmi les myofibroblastes, les fibroblastes, les myocytes, les cardiomyocytes et les cellules musculaires (y compris les mélanges de deux, trois, quatre ou cinq de ces types cellulaires) et progéniteurs pour ces cellules et les cellules capables de se différencier en ces cellules.

La différenciation des cellules souches, notamment mésenchymateuses, peut notamment comprendre une mise en contact de ces cellules avec au moins un agent choisi parmi TGFbêta (y compris TGFbêta1), CTGF, EGF et NUX1, un stress mécanique, ou une stimulation électrique. Une activité paracrine du tissu contractile peut être induite par mise en contact de cellules de ce tissu avec au moins un agent choisi parmi IFNgamma, TNFalpha et IL1bêta et/ou par application d'une hypoxie transitoire sur ces cellules.

Le support (solide optionnellement poreux) peut présenter une complaisance d'élasticité de 0,1 à 30 kPa⁻¹. Par exemple, les supports de contractilité sans cellule peuvent avoir une complaisance d'élasticité de 0,4 kPa⁻¹ et de 1.5 kPa⁻¹ après colonisation cellulaire.

### BRÈVE DESCRIPTION DES FIGURES

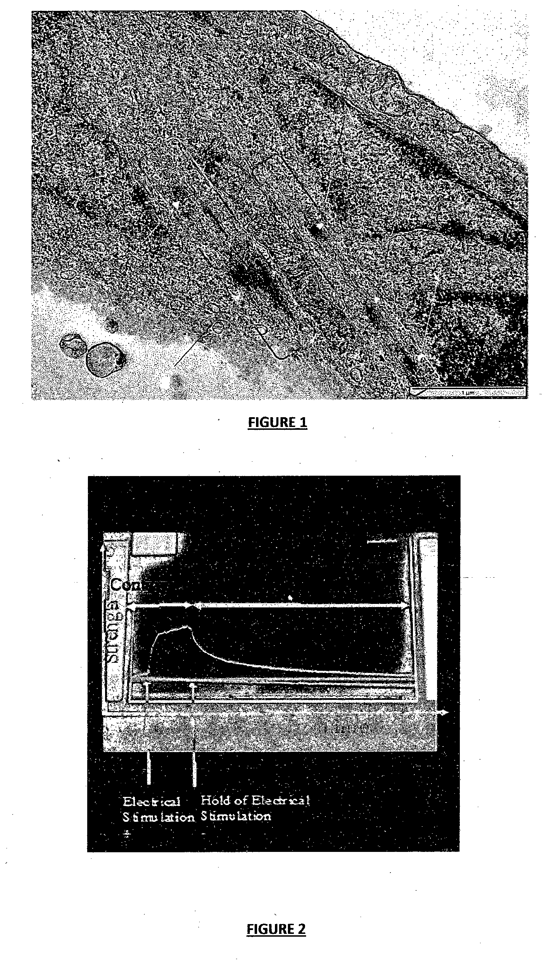

La **Figure 1** montre une observation au microscope électronique d'un biomatériau de la demande (matrice de collagène réticulée à titre de support solide poreux et MSC différenciées par contact avec du lysat plaquettaire ; *cf.* exemple 1 ci-dessous). Les rectangles tracés délimitent les zones identifiables de protéines filaments contractiles. Les flèches montrent une interruption régulière de ces filaments par des denses bodies ou peut être un sarcomère. Le biomatériau s'est donc organisé en structure contractile. À notre connaissance, des bandes de stress et une organisation terminale de l'appareil contractile n'ont jamais été mises en évidence avec des MSC placées en culture standard 2D.
La **Figure 2** illustre le résultat de contractilité présenté par un biomatériau de la demande (matrice de collagène réticulée par DHT ; MSC différenciées en myofibroblastes à au moins 80%). Les cinétiques de contraction et de relaxation sont proches de celles du tissu placentaire (*cf.* exemple 2 ci-dessous).

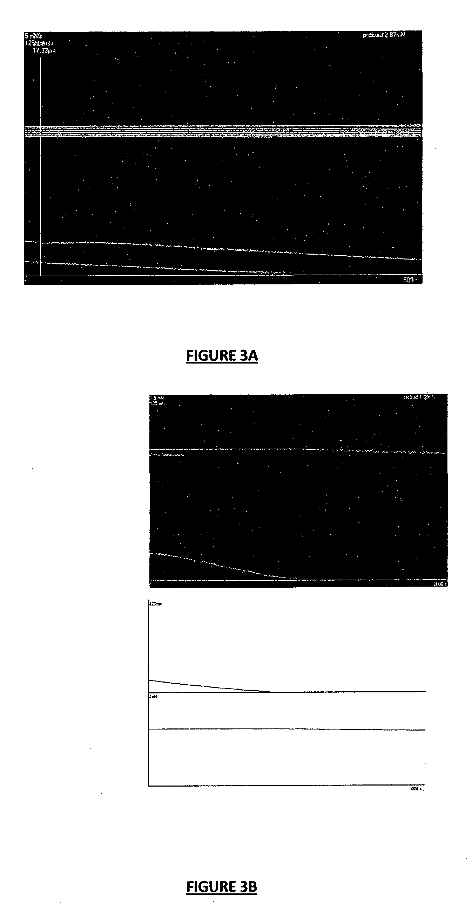

Les **Figures 3A et 3B** illustrent le fait que la propriété de relaxation d'un biomatériau de la demande est bien un phénomène actif, et non un phénomène passif (relaxation avec allongement [courbes du haut en Figure 3A et 3B] et avec diminution de la force développée [courbes du bas en Figures 3A et 3B]). Figure 3A ; cas d'un biomatériau avec une matrice de collagène réticulée par DHT et avec des molécules de peptide RGD, sous stimulation électrique en tétanus et avec inhibition de la relaxation par RISORDAN. Figure 3B : cas d'un biomatériau avec une matrice de collagène réticulée par DHT, sous stimulation par KCI 0,05 M, et avec une inhibition par BDM *(cf.* exemple 2 ci-dessous).

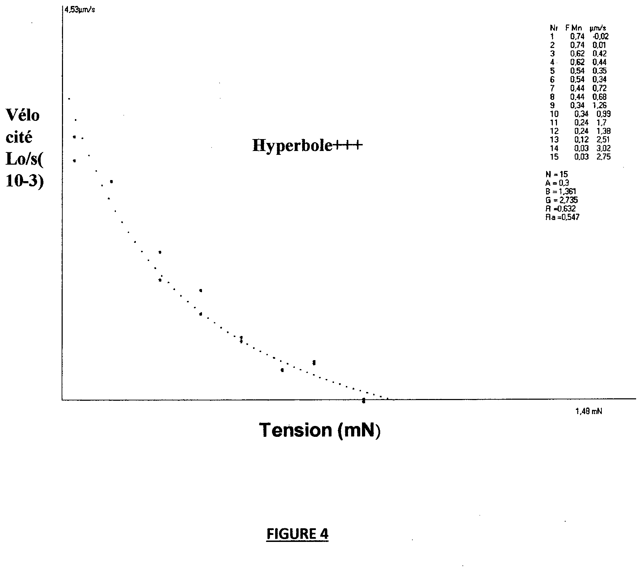

La **Figure 4** montre qu'une courbe de Hill a pu être établie pour un biomatériau de la demande (biomatériau avec matrice de collagène réticulée par DHT, et conservé à une température supérieure à 0°C -en l'occurrence de 4°C environ-). La courbe de Hill (hyperbole) est spécifique des tissus contractiles (abscisses : tension (mN) ; ordonnées vélocité Lo/s(10⁻³) ; *cf.* exemple 2 ci-dessous).

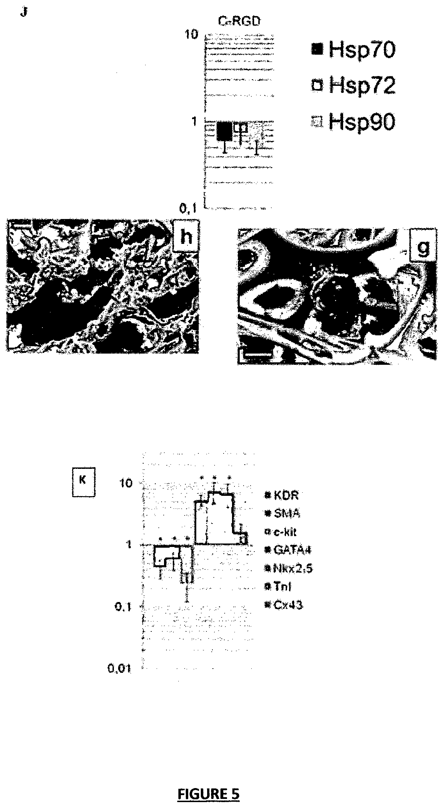

La **Figure 5** montre la migration des cellules des cardiosphères dans les supports (changement Vs. IICSps (échelle log)), les cellules ne restent pas sous forme de cluster *(cf.* g) mais migrent et se répartissent dans tout le support *(cf.* h). Une induction des marqueurs cardiaques et une diminution des protéines du stress Hsp70, Hsp72, Hsp90 a été observée. A également été observée une activation de Akt dans les cellules MSC.

### DESCRIPTION DÉTAILLÉE

L'invention est exposée dans le jeu de revendications joint et concerne un procédé de production d'un biomatériau cellulaire contractile, qui comprend les étapes suivantes :
- placer des cellules souches mésenchymateuses à l'intérieur d'un support tridimensionnel, ledit support présentant des pores permettant la diffusion des nutriments, et la colonisation cellulaire in vitro, et in vivo,
- induire la différenciation des cellules souches mésenchymateuses multipotentes en cellules contractiles, choisies parmi les myofibroblastes, les myocytes, les cardiomyocytes, ou les cellules musculaires striées, dans un support tridimensionnel à complaisance d'élasticité qui permet la formation d'un tissu contractile par la mise en contact desdites cellules souches mésenchymateuses avec un agent favorisant la différenciation choisi parmi TGFbêta, CTGF, EGF, NUX1 et le lysat plaquettaire, et
- induire une activité paracrine desdites cellules contractiles, l'activité paracrine induite comprenant de plus une activité d'immunosuppression de cellules T et/ou activation de l'indoleamine 2,3-dioxygenase (IDO), ladite activité paracrine est induite par la mise en contact de ces cellules contractiles avec au moins un agent inducteur d'activité paracrine choisi parmi IFNgamma, TNFalpha et IL1bêta, et/ou l'application d'une hypoxie transitoire sur ces cellules contractiles,
dans lequel
ledit support tridimensionnel présente une complaisance d'élasticité sans cellule de 0,1 à 30kPa⁻¹, plus particulièrement de 0.4 à 20 kPa⁻¹, plus particulièrement de 0.5 à 15 kPa⁻¹, plus particulièrement de 0.5 à 5 kPa⁻¹,
ledit support contenant les cellules contractiles ainsi produit est un biomatériau cellulaire contractile adapté à la thérapie régénérative du tissu myocardique.

En outre, la présente demande décrit un biomatériau contractile, plus particulièrement un (bio)matériau cellulaire contractile.

Ce biomatériau est notamment adapté à la thérapie régénérative (ou cicatricielle) du tissu myocardique, plus particulièrement à une thérapie régénérative (ou cicatricielle) appliquée à un tissu myocardique ischémique et inflammatoire, plus particulièrement à la cicatrisation myofibroblastique *post*-infarctus.

Les moyens de la demande peuvent notamment
- limiter voire éviter le remodelage ventriculaire ; et/ou
- diminuer la taille de l'infarctus myocardique ou favoriser la régénération myocardique ; et/ou
- favoriser ou augmenter l'angiogenèse au niveau de la zone infarcie.

De manière particulièrement notable, les moyens de la demande peuvent conduire à une amélioration de la contractilité myocardique.

Le biomatériau comprend des cellules et un support, qui est un support de contractilité. Ledit support est un support tridimensionnel. Le support peut être un support solide, notamment un support solide poreux, plus particulièrement un support solide poreux tridimensionnel.

Ledit support peut par exemple être une éponge (par exemple une éponge réticulée par DHT) ou une mousse. Ledit support peut, par exemple, être un gel ou un hydrogel.

Le support peut être associé à un agent.

Le support peut être stimulé physiquement

Le support peut être injectable ou administrable par voie mini-invasive. Il peut avoir une mémoire de forme. Il peut être obtenu par 3D printing. Le support peut être réticulé chimiquement, physiquement, ou enzymatiquement. Le support peut être orienté.

Le support peut par exemple être un support à base de collagène (collagène naturel ou non, purifié ou non), éventuellement chimiquement modifié par traitement chimique, par la chaleur, ou par traitement à la gélatine. Le support peut être modifié avec des molécules d'adhésions liées de manière covalente.

Les cellules sont obtenues par différenciation de cellules souches. Elles forment un tissu cellulaire et sont présentes à l'intérieur de la structure moléculaire (poreuse) du support (solide), c'est-à-dire dans les pores du support (solide poreux). Le support, plus particulièrement le support solide optionnellement poreux, peut donc être perçu comme un support 3D (par opposition à la culture cellulaire classique, qui est une culture 2D).

Le biomatériau est contractile, c'est-à-dire qu'il a des propriétés de contraction et de relaxation. En effet, le tissu cellulaire qui est produit par différenciation des cellules souches est contractile, et le support (solide) (poreux) qui contient ce tissu cellulaire présente une complaisance d'élasticité qui permet à cette contractilité de s'exprimer.

Le biomatériau (c'est-à-dire le tissu cellulaire qu'il contient) présente en outre une activité paracrine inductible. L'activité paracrine inductible ou induite comprend notamment une activité d'immuno-modulation, notamment d'immunosuppression de cellules T.

De manière remarquable, le biomatériau peut être conservé hors congélation. En effet, il peut être conservé à une température supérieure à 0°C et conserver ses propriétés de contractilité. Par exemple, il présente toujours des propriétés de contractilité, et optionnellement d'activité paracrine inductible, après conservation pendant 5 jours à une température supérieure à 0°C mais inférieure à 10°C.

Le biomatériau de la demande ne requiert donc pas d'être congelé et, de ce fait, peut donc être très rapidement disponible et fonctionnel : à la sortie de son récipient de stockage, le biomatériau de la demande est en fait immédiatement administrable ou transplantable. Le sujet ou patient peut le recevoir sans délai de décongélation, changement de milieu, ou culture. Il peut être même utilisé à température ambiante, pas nécessairement à 37°C. Les cellules étant adhérentes au support et le support ayant une porosité importante, il y a une libre diffusion des milieux à travers le support qui va permettre le changement de milieu sans nécessiter de faire des centrifugations itératives pour récupérer normalement des cellules en suspensions.

Lorsqu'il est placé sur un tissu myocardique ischémique et inflammatoire, le biomatériau de la demande agit comme un pansement cicatriciel. Il y favorise la régénération myocardique. Il peut également y favoriser l'angiogenèse.

Le biomatériau de la demande permet de limiter voire d'éviter le remodelage ventriculaire post-infarctus. Il peut en outre diminuer la taille de la zone infarcie. Le biomatériau peut éventuellement préparer le ventricule à une thérapie cellulaire, ou autre visant à traiter un myocarde altéré. Plusieurs biomatériaux peuvent être utilisés simultanément ou dans le temps. Les biomatériaux peuvent être empilés ou utilisés sous forme de « sandwich ». Ils peuvent être appliqués dans un site extracardiaque avant d'être appliqués en libre ou en greffon vascularisé sur la zone infarcie. Un des sites peut être la cavité péritonéale avec notamment l'épiploon, des muscles. Le biomatériau peut être couvert ou protégé sur le myocarde par un dispositif ou éventuellement un patch péricardique.

De manière particulièrement notable, le biomatériau de la demande peut conduire à une préservation ou une amélioration de la contractilité myocardique.

La demande décrit un procédé de production de ce biomatériau.

Le procédé de la demande comprend le fait d'induire la différenciation de cellules souches en cellules contractiles, plus particulièrement en cellules qui forment (entre elles) (et avec le support) un tissu contractile.

Le procédé de la demande peut comprendre le fait d'induire la différenciation de cellules souches en cellules contractiles préférentiellement dans le support tridimensionnel de contractilité qui va aider à cette différenciation. Ce support de contractilité avec une complaisance d'élasticité déterminée va permettre de développer un tissu contractile avec apparition de force et de raccourcissement de la structure formée. Cette contractilité peut être spontanée ou provoquée. Elle peut ne pas être permanente. Pour avoir un tissu contractile il est important que les cellules dans le support soient contractiles ou aient la capacité de le devenir. Ces cellules contractiles doivent être connectées entre elles et avec le support. Les monomères/polymères du support peuvent être connectés. La complaisance d'élasticité du support ne doit pas être nulle. Elle doit être compatible avec une déformation par le contingent cellulaire associé. Il ne s'agit pas d'une émulsion où les éléments cellulaires et contractiles ne sont pas connectés et la complaisance d'élasticité est nulle. Si la complaisance d'élasticité est trop importante du tissu osseux cartilagineux sera produit. Si la complaisance d'élasticité est trop basse, on n'obtiendra pas du tissu contractile (et les fibroblastes par exemple ne se différencieront pas en myofibroblastes).

Les cellules souches peuvent être des cellules souches multipotentes et/ou des cellules souches pluripotentes, notamment des cellules souches adultes (non-embryonnaires), notamment des cellules souches cardiaques (CSC) ou des cellules souches mésenchymateuses, plus particulièrement des cellules souches mésenchymateuses. Il s'agit plus particulièrement de cellules humaines. Les cellules peuvent éventuellement être modifiées génétiquement. Les cellules souches peuvent provenir de la reprogrammation génétique de cellules différentiées. Les cellules peuvent être des cellules pluripotentes induite (« IPS »). Les cellules souches peuvent provenir de banque de cellules utilisables en clinique. Les cellules peuvent être produite sous la forme de cluster cellulaire. Les cellules peuvent être fusionnées.

Les cellules souches mésenchymateuses sont souvent dérivées du mésoderme et également d'une trans-différenciation de l'endothélium ou épithélium vers le mésenchyme.

Les cellules souches, notamment les cellules souches mésenchymateuses, peuvent provenir de toute origine que la personne du métier juge appropriée, par exemple d'un tissu adipeux, de la moelle osseuse, d'un tissu de soutien d'un organe, d'un os, d'un cartilage, ou d'un muscle, du tissu cardiaque et dérivés.

Les cellules souches, notamment les cellules souches mésenchymateuses, peuvent être autologues ou hétérologues. Les cellules peuvent être autologues ou allogéniques.

Elles peuvent provenir de tissu humain non utilisé ou éliminé pour une autre cause également de tissu prélevé chez une personne décédée récemment.

Dans la présente demande, à moins que cela ne soit autrement spécifié, ou que le contexte n'en dicte autrement, tous les termes ont leur sens habituel dans le(s) domaine(s) concerné(s).

Par exemple, le terme « cellules souches mésenchymateuses » (*Mesenchymal Stem Cells* ou *Mesenchymal Stromal Cells* ou MSC) se réfère en général aux cellules telles que définies dans Dominici et al. 2006 (Cytotherapy 8(4) : 315-317), c'est-à-dire à des cellules adhérentes au plastique (après 24h de contact avec la surface du plastique d'une boîte culture par exemple), qui expriment CD105, CD73 et CD90, qui n'expriment pas CD45, CD34, CD14 (ou C11b), CD79a (ou CD19), et HLA-DR. Les MSC peuvent en outre être caractérisées par le fait qu'elles peuvent se différencier en ostéoblastes, adipocytes et chondroblastes.

Le fait d'induire la différenciation de cellules souches, notamment de cellules souches mésenchymateuses, en cellules contractiles, plus particulièrement en cellules formant un tissu contractile, peut notamment comprendre la mise en contact des cellules souches, notamment des cellules souches mésenchymateuses, avec au moins un agent favorisant la différenciation choisi parmi
TGFbêta *(Tumor Growth Factor* bêta) (plus particulièrement au moins TGFbêta1),
CTGF (*Connective Tissue Growth Factor*),
EGF (*Epidermal Growth Factor*) et
RUNX1 (*Runt-related transcription factor 1*),
les régulateurs de transcription et
un stress mécanique,

plus particulièrement parmi TGFbêta, CTGF, EGF et RUNX1,
plus particulièrement avec au moins TGFbêta (plus particulièrement au moins TG Fbêta1), et
plus particulièrement avec au moins TGFbêta (plus particulièrement au moins TGFbêta1) et optionnellement avec au moins un autre agent de différenciation choisi parmi CTGF, EGF et RUNX1. Le lysat plaquettaire est un produit qui contient TGFbêta (plus particulièrement TGFbêta1) et/ou CTGF et/ou EGF et/ou RUNX1, plus particulièrement au moins TGFbêta (et optionnellement CTGF et/ou EGF et/ou RUNX1), plus particulièrement au moins TGFbêta1 (et optionnellement CTGF et/ou EGF et/ou RUNX1). Le fait d'induire la différenciation de cellules souches, notamment de cellules souches mésenchymateuses, en cellules formant un tissu contractile peut donc comprendre la mise en contact des cellules souches, notamment des cellules souches mésenchymateuses, avec (au moins) du lysat plaquettaire.

Les cellules contractiles (ou les cellules qui forment le tissu contractile) peuvent être des cellules différenciées, ou un mélange de cellules différenciées et de cellules souches (lequel mélange comprenant au moins 50%, notamment au moins 80%, de cellules différenciées).

Les cellules contractiles (ou les cellules (différenciées) formant le tissu contractile) peuvent comprendre ou être des cellules choisies parmi les myofibroblastes, les fibroblastes, les myocytes, les cardiomyocytes, les cellules musculaires (y compris les mélanges de deux, trois, quatre ou cinq de ces types cellulaires), plus particulièrement des cellules choisies parmi les myofibroblates et les fibroblastes (y compris les mélanges de myofibroblastes et de fibroblastes). Plus particulièrement, les cellules (différenciées) formant le tissu contractile peuvent comprendre ou être des myofibroblates.

Plus particulièrement, cellules contractiles (ou les cellules (différenciées) formant le tissu contractile) peuvent comprendre ou être des cellules contractiles.

Le procédé comprend en outre la production d'un support (solide) (poreux) qui contient ce tissu contractile et permet le développement de la contractilité. L'expression « un support (solide) (poreux) qui contient un tissu contractile » signifie que tout ou partie d'un tissu contractile se trouve à l'intérieur du support (solide) (poreux), plus particulièrement dans les pores du support (solide) (poreux). Les cellules dans le support (poreux) vont adhérer aux parois de la structure et également empilement de cellules, l'ensemble connecté va permettre une activité synchrone contractile efficace.

Les cellules souches, notamment les cellules souches mésenchymateuses, ont une activité paracrine inductible. Le tissu contractile obtenu dans le support compliant comprend des cellules qui se sont différenciées (généralement, au moins 80% des cellules souches, notamment des cellules souches mésenchymateuses, se sont différenciées).

Or, de manière remarquable, le tissu contractile obtenu dans le support (solide) présente toujours une activité paracrine qui est inductible et même amplifiée avec apparition de facteurs spécifiques alors que les cellules ne sont plus en culture 2D mais 3D, et que les cellules se sont majoritairement différentiées en cellules contractiles.

Cette activité paracrine inductible ou induite comprend une activité (paracrine) d'immuno-modulation, notamment d'immunosuppression, plus particulièrement d'immunosuppression de cellules T. L'activité immunosuppressive des MSC sur les lymphocytes T n'est pas due à un contact direct entre les MSC et les lymphocytes T. La fonction immunosuppressive des MSC passe par l'activation de l'indoleamine 2,3-dioxygenase (IDO) qui va induire une consommation du tryptophane du milieu de culture qui est en grande partie responsable sur l'effet antiprolifératif sur les lymphocytes T. Cette activation va s'accompagner également de la synthèse de très nombreux facteurs de croissance qui vont être relargués dans le milieu. Les facteurs libérés sont par exemple le facteur de croissance épidermique (*epidermal growth factor* ou EGF), *platelet derived growth factor* (PDGF), le facteur de croissance des plaquettes sanguines (*vascular endothelial growth factor* ou VEGF), le facteur de croissance transformant (*transforming growth factor* ou TGFbêta), le facteur de croissance des hépatocytes (*hepatocyte growth factor* ou HGF), le facteur de croissance ressemblant à l'insuline (*insulin growth factor* tel que IGF1), l'angiopoïétine 1 (Ang1), le facteur de croissance des kératinocytes (*keratinocyte growth factor* ou KGF), le facteur 1 dérivé du stroma (*stroma derived factor 1* ou SDF1), la protéine de type follistatine 1 *(Follistatin-like* 1/TSC36 ou FSLT1), l'Interleukine 10 (Il-10), la PGE2, des métalloprotéinases et des régulateurs comme MMP2, MMP9, T1MP1, T1MP2. La majorité de ces cytokines ont leurs sécrétions amplifiées dans la structure 3D à complaisance d'élasticité. Le support de contractilité augmente la signalisation IL-1 et TGFbêta. IL-1 est le premier médiateur mis en route dans l'inflammation après infarctus et est responsable du recrutement initial des cellules inflammatoires PNN (polynucléaires neutrophiles) dans les toutes premières heures. De nombreux facteurs immunomodulateurs sont également relargués par les MSC activées comme IDO, semaphorine-3A, B7-H4, LIF, TSG6, galectine, HO-1, Il-6, IL-10, TGFbêta, PGE2, PD-L1/2. Certains facteurs paracrines libérés nécessitent une structure 3D avec complaisance d'élasticité comme le TSG6 et CXCR4. Le TSG6 est connu pour avoir un rôle important dans l'infarctus. Dans les supports 3D à complaisance d'élasticité la production de PGE2 est particulièrement augmentée. L'activation des cellules dans du support par l'interféron gamma va augmenter la synthèse de TGFbêta, PGE2, BMP2, facteur H (inhibiteur du complément), Gal9. Le facteur H est très important car dans la zone infarcie il y a beaucoup de complément qui va conduire à la lyse locale des cellules. Le facteur H va donc protéger les cellules de la lyse par le complément et donc améliorer leur survie. Les cellules décongelées ont une diminution du facteur H est donc sont particulièrement sensible à la lyse par le complément. La préparation de la demande est donc très supérieure. L'IL-10 est connue pour réduire la réponse inflammatoire après infarctus. L'IDO et la PGE2 favorisent la transformation des macrophages de type M1 vers des macrophages de type M2 après infarctus. Pour ce qui est de la lyse du collagène naturel non dénaturé les MSC en culture 2D ne peuvent pas lyser le collagène car les activités MMP14, MMP1, MMP8 ou MMP13 ne sont pas présentes. L'activité MMP14 est une métalloprotéinase membranaire qui reste confinée à la cellule. Comme les MSC restent dans le support cette activité va donc rester contenue dans le support. L'activité MMP14 peut permettre de lyser effectivement le collagène naturel qui est particulièrement surreprésenté après infarctus dans la zone d'escarre. L'activité MMP14 permet également de modifier les activités des métalloprotéinases MMP2 et MMP9 solubles qui seront une fois activées capables de lyser le collagène naturel. Ces facteurs pourront alors modifier le collagène naturel environnant.

Il a été montré que les facteurs paracrines libérés par les MSC *in vitro* immunosuppressives sont des facteurs qui vont promouvoir la régénération tissulaire *in vivo.* Les facteurs paracrines libérés par les MSC immunosuppressive vont par exemple accélérer la transformation des macrophages M1 vers des macrophages de type M2 dans l'infarctus.

Ces facteurs peuvent être éventuellement relargués dans des vésicules ou dans des structures membranaires plus complexes de types « exosomes ».

L'activité paracrine inductible ou induite peut ainsi comprendre une activité (paracrine) d'inhibition de la prolifération des lymphocytes T.

Plus particulièrement, l'activité paracrine inductible ou induite est une activité (paracrine) qui favorise l'immuno-modulation (notamment l'immunosuppression) et qui favorise la génération cellulaire et/ou l'angiogenèse. Plus particulièrement, l'activité paracrine inductible ou induite est une activité (paracrine) qui favorise l'immunosuppression de cellules T.

Le procédé de la demande peut donc en outre comprendre le fait d'induire une activité paracrine desdites cellules contractiles (ou dudit tissu contractile, c'est-à-dire d'induire une activité paracrine chez des cellules de ce tissu contractile).

Le fait d'induire une activité paracrine desdites cellules contractiles (ou de cellules dudit tissu contractile) peut notamment comprendre :
- la mise en contact de cellules de ces cellules contractiles (ou de ce tissu contractile) avec au moins un agent inducteur d'activité paracrine choisi parmi
   IFNgamma (interféron gamma),
   TNFalpha *(Tumor Necrosis Factor* alpha) et
   IL1bêta (interleukine 1 bêta),
      plus particulièrement avec au moins de l'IFNgamma,
      plus particulièrement avec de l'IFNgamma et optionnellement au moins un autre agent inducteur de différenciation choisi parmi TNFalpha et IL1bêta, et/ou
- l'application d'une hypoxie transitoire sur lesdites cellules contractiles (ou sur des cellules dudit tissu contractile), par exemple une hypoxie inférieure à 5% pendant 1 ou 2 jours.

La mise en contact avec l'IFNgamma peut par exemple être faite à hauteur de plus de 10 UI/mL, plus particulièrement de 50 à 1000 UI/mL, plus particulièrement de 50 à 500 UI/mL d'IFNgamma. Alternativement ou complémentairement à l'induction d'une activité paracrine du tissu contractile, le procédé de la demande peut comprendre l'induction d'une activité paracrine sur les cellules souches, plus particulièrement sur les cellules souches mésenchymateuses, avant qu'elles ne soient différenciées en cellules formant un tissu contractile. Ainsi, avant d'induire la différenciation des cellules souches, plus particulièrement des cellules souches mésenchymateuses, en cellules contractiles, plus particulièrement en cellules formant un tissu contractile, le procédé de la demande peut comprendre (alternativement ou complémentairement à l'induction d'une activité paracrine du tissu contractile) :
- la mise en contact desdites cellules souches, plus particulièrement desdites cellules mésenchymateuses, avec au moins un agent inducteur d'activité paracrine choisi parmi IFNgamma (interféron gamma), TNFalpha *(Tumor Necrosis Factor* alpha) et IL1bêta (interleukine 1 bêta), plus particulièrement avec au moins de l'IFNgamma, plus particulièrement avec de l'IFNgamma et optionnellement au moins un autre agent inducteur de différenciation choisi parmi TNFalpha et IL1bêta, et/ou
- l'application d'une hypoxie transitoire sur lesdites cellules souches, plus particulièrement desdites cellules mésenchymateuses (par exemple une hypoxie inférieure à 5% pendant 1 ou 2 jours).

Complémentairement ou alternativement au fait d'être poreux, le support (solide) mis en oeuvre peut t présenter une complaisance d'élasticité de 0,1 à 30 kPa⁻¹, plus particulièrement de 0.4 à 20 kPa⁻¹, plus particulièrement de 0.5 à 15 kPa⁻¹, plus particulièrement de 0.5 à 5 kPa⁻¹ (complaisance du support solide (poreux) avec ou sans cellule, plus particulièrement sans cellule).

La complaisance d'élasticité du support peut être obtenue après colonisation cellulaire et réticulation. La colonisation cellulaire et la réticulation augmentent la complaisance d'élasticité du support.

Au sens de la science des matériaux, la complaisance d'élasticité (*compliance* en anglais) est une grandeur qui caractérise le comportement élastique d'un matériau. Une complaisance d'élasticité est définie comme l'inverse d'un module élastique, et s'exprime en Pa⁻¹. Schématiquement, la complaisance d'élasticité peut être vue comme le degré auquel un contenant peut subir une pression ou force sans se rompre.

Le procédé tel que décrit dans la demande peut ainsi être défini comme étant un procédé de production d'un biomatériau cellulaire contractile, qui comprend :
- le fait d'induire la différenciation de cellules souches mésenchymateuses multipotentes en cellules contractiles dans un support de contractilité qui est un support (solide) (tridimensionnel), et le fait d'induire une activité paracrine desdites cellules contractiles, l'activité paracrine induite comprenant en outre une activité d'immunosuppression de cellules T et/ou activation de l'indoleamine 2,3-dioxygenase (IDO),
ledit procédé comprend en outre
- le fait d'induire la différenciation de cellules souches mésenchymateuses multipotentes en cellules contractiles comprend la mise en contact des cellules souches mésenchymateuses avec au moins un agent favorisant la différenciation choisi parmi TGFbêta, CTGF, EGF, NUX1, les régulateurs de transcription et un stress mécanique et une stimulation électrique, plus particulièrement parmi TGFbêta, CTGF, EGF et NUX1,
- ledit support (solide) tridimentionnel présente une complaisance d'élasticité de 0,1 à 30 kPa⁻¹, et
- le fait d'induire une activité paracrine desdites cellules contractiles comprend la mise en contact de ces cellules contractiles avec au moins un agent inducteur d'activité paracrine choisi parmi IFNgamma, TNFalpha et IL1bêta, et/ou l'application d'une hypoxie transitoire sur des cellules de ce tissu contractile,
le support (solide) de contractilité contenant les cellules contractiles ainsi produit étant un biomatériau cellulaire contractile adapté à la thérapie régénérative du tissu myocardique.

Plus particulièrement, le procédé de la demande peut ainsi être défini comme étant un procédé de production d'un (bio)matériau cellulaire contractile adapté à la thérapie régénérative du tissu myocardique, qui comprend :
le fait d'induire la différenciation de cellules souches mésenchymateuses en cellules contractiles et de produire un support (solide) (poreux) qui contient ces cellules contractiles, et
le fait d'induire une activité paracrine chez ces cellules contractiles, l'activité paracrine induite comprenant une activité d'immunosuppression de cellules T,
ledit procédé comprend en outre
l'induction de la différenciation des cellules souches mésenchymateuses en cellules contractiles comprend la mise en contact des cellules souches mésenchymateuses avec au moins un agent favorisant la différenciation choisi parmi TGFbêta (plus particulièrement avec au moins TGFbêta1), CTGF, EGF et RUNX1, plus particulièrement avec au moins TGFbêta (plus particulièrement avec au moins TGFbêta1), plus particulièrement avec au moins TGFbêta (plus particulièrement avec au moins TGFbêta1) et optionnellement au moins un autre agent favorisant la différenciation choisi parmi CTGF, EGF, RUNX1, les régulateurs de transcription et un stress mécanique, plus particulièrement parmi CTGF, EGF et RUNX1,
les cellules contractiles comprennent ou sont des cellules choisies parmi les myofibroblastes, les fibroblastes, les myocytes, les cardiomyocytes, les cellules musculaires (y compris les mélanges de deux, trois, quatre ou cinq de ces types cellulaires), plus particulièrement comprennent ou sont des cellules choisies parmi les myofibroblates et les fibroblastes, plus particulièrement comprennent ou sont des myofibroblates,
ledit support (solide) (poreux) présente une complaisance d'élasticité de 0,1 à 30 kPa⁻¹, et
l'induction de l'activité paracrine chez les cellules contractiles comprend
   la mise en contact de ces cellules contractiles avec au moins un agent inducteur d'activité paracrine choisi parmi IFNgamma, TNFalpha et IL1bêta, plus particulièrement avec au moins IFNgamma et optionnellement avec au moins un autre agent inducteur d'activité paracrine choisi parmi TNFalpha et IL1bêta, et/ou
   l'application d'une hypoxie transitoire sur ces cellules contractiles.

Plus particulièrement, le procédé tel que décrit dans la demande peut ainsi être défini comme étant un procédé de production d'un (bio)matériau cellulaire contractile adapté à la thérapie régénérative du tissu myocardique après infarctus, et en ce que les cellules contractiles sont choisies parmi les myofibroblastes, les fibroblastes, les myocytes, les cardiomyocytes et les cellules musculaires et les progéniteurs de ces cellules, et les cellules capables de se différencier en ces cellules.

Plus particulièrement, ledit procédé tel que décrit dans la demande comprend les étapes:
- induire la différenciation des cellules souches mésenchymateuses en cellules contractiles qui comprend la mise en contact des cellules souches mésenchymateuses avec TGFbêta, plus particulièrement avec TGFbêta1, et
- induire une activité paracrine desdites cellules contractiles qui comprend la mise en contact des cellules de ce tissu avec IFNgamma.

Les cellules contractiles décritent ici forment un tissu contractile dans le support (solide) (poreux). Le support (solide) (poreux) contenant le tissu contractile ainsi produit est un biomatériau cellulaire contractile adapté à la thérapie régénérative de tissus, notamment de tissus humains, tels qu'un tissu musculaire, un tissu humain non contractile, un tissu cardiaque, plus particulièrement un tissu myocardique humain.

Plus particulièrement, le procédé tel que décrit dans la demande peut être défini comme étant un procédé de production d'un (bio)matériau cellulaire contractile adapté à la thérapie régénérative du tissu myocardique, qui comprend le fait d'induire la différenciation de cellules souches mésenchymateuses en cellules contractiles qui dans le support à complaisance d'élasticité vont former une structure contractile (notamment, un tissu contractile), et le fait d'induire une activité paracrine chez ces cellules contractiles, l'activité paracrine induite comprenant une activité d'immunosuppression de cellules T, ledit procédé tel que décrit dans la demande comprend l'induction de la différenciation des cellules souches mésenchymateuses en cellules contractiles comprend la mise en contact des cellules souches mésenchymateuses avec (au moins) TGFbêta plus particulièrement avec (au moins) TGFbêta1 (et optionnellement au moins un autre agent favorisant la différenciation choisi parmi CTGF, EGF et RUNX1),
les cellules contractiles comprennent ou sont des cellules choisies parmi les myofibroblastes, les fibroblastes, les myocytes, les cardiomyocytes, les cellules musculaires et leurs progéniteurs, une préparation de cellules souches contenant des cellules mésenchymateuses, comme des « cardiosphères » par exemple, pour ces cellules (y compris les mélanges de deux, trois, quatre ou cinq de ces types cellulaires), plus particulièrement comprennent ou sont des cellules choisies parmi les myofibroblates et les fibroblastes, plus particulièrement comprennent ou sont des myofibroblates,
ledit support (solide) (poreux) présente une complaisance d'élasticité de 0,1 à 30 kPa⁻¹, et l'induction de l'activité paracrine chez les cellules contractiles comprend la mise en contact de ces cellules contractiles avec (au moins) IFNgamma (et optionnellement avec au moins un autre agent inducteur d'activité paracrine choisi parmi TNFalpha et IL1bêta).

Selon un mode de réalisation, on place ou implante les cellules souches (notamment les cellules souches mésenchymateuses) dans le support (solide) (poreux) (notamment dans les pores de ce support (solide) (poreux)) avant d'induire leur différenciation en cellules formant un tissu contractile. Toutes les cellules souches (notamment toutes les cellules souches mésenchymateuses), ou au moins 50% d'entre elles ou au moins 60% d'entre elles ou au moins 80% d'entre elles, peuvent être placées ou implantées (ou contenues) à l'intérieur de la structure (moléculaire) poreuse du support (solide).

Le procédé tel que décrit dans la demande peut alors comprendre le fait de placer ou implanter des cellules souches (mésenchymateuses) dans le support (solide) (poreux) (plus particulièrement dans les pores du support (solide) (poreux)), puis d'induire la différenciation de ces cellules souches (mésenchymateuses) en cellules formant un tissu contractile.

L'induction de la différenciation de cellules souches (mésenchymateuses) en cellules formant un tissu contractile et la production d'un support (solide) (poreux) qui contient ce tissu contractile peuvent ainsi être réalisées :
en produisant un support (solide) (poreux) dans lequel (dans les pores duquel) des cellules souches (mésenchymateuses) sont contenues et
en induisant la différenciation de tout ou partie (au moins 50%, plus particulièrement au moins 80%) de ces cellules souches (mésenchymateuses) en cellules qui forment un tissu contractile (à l'intérieur de la structure poreuse du support (solide)).

Les cellules qui forment le tissu contractile peuvent notamment être liées entre elles par des jonctions intercellulaires. Les jonctions intercellulaires peuvent notamment être ou comprendre des jonctions intercellulaires à connexines (c'est-à-dire des jonctions intercellulaires dont les protéines de jonction comprennent une ou des connexines). Les jonctions intercellulaires peuvent plus particulièrement être ou comprendre des jonctions intercellulaires communicantes, plus particulièrement des jonctions intercellulaires communicantes à connexons.

Le tissu contractile est, ou se trouve, fixé au support. Ledit tissu contractile est lié au support (solide) (poreux), par exemple par des jonctions d'ancrage. Les jonctions d'ancrage peuvent notamment être des molécules d'ancrage. Les molécules d'ancrage peuvent notamment comprendre des protéines d'ancrage, plus particulièrement au moins une protéine transmembranaire relié au cytosquelette et à l'appareil contractile de la cellule. Les jonctions d'ancrage peuvent notamment être des molécules d'ancrage, notamment des protéines d'ancrage, qui comprennent au moins une intégrine qui sont des mécanorécepteurs et donc vont permettre de déterminer la complaisance d'élasticité du support. La stimulation des récepteurs va induire une augmentation des connexions des cellules entres elles et également améliorer la survie des cellules et favoriser la différenciation en cellules contractiles et une organisation de l'appareil contractiles en fonction de la localisation de cette interaction en la renforçant également.

Plus particulièrement, le procédé tel que décrit dans la demande comprend des cellules contractiles qui forment un tissu contractile, et un tissu contractile qui est lié au support de contractilité par des molécules d'ancrage qui comprennent au moins une intégrine.

La différenciation des cellules souches, notamment des cellules souches mésenchymateuses, en cellules formant un tissu contractile peut être induite jusqu'à détection d'un marqueur cellulaire de différenciation, ledit marqueur cellulaire étant par exemple choisi parmi l'alphaSMA (*alphaSmoothMuscleActine*) et la mysosine (plus particulièrement la mysosine de type II). Ledit marqueur cellulaire est plus particulièrement l'alphaSMA. Le tissu contractile contient alors des cellules qui présentent au moins le marqueur alphaSMA, plus particulièrement le marqueur alphaSMA et la myosine (plus particulièrement la mysosine de type II). Au niveau des cellules contractiles dans les supports de contractilité, l'alphaSMA va être associée aux bandes de contractilité elles même en regard de l'interaction de surface intégrinique.

Les cellules formant un tissu contractile peuvent être ou comprendre des myofibroblastes, des fibroblastes, des myocytes, des cardiomyocytes, des cellules musculaires (notamment des cellules musculaires squelettiques, des cellules musculaires lisses ou des cellules musculaires striées). Les cellules formant un tissu contractile peuvent être un mélange de plusieurs de ces types cellulaires, par exemple un mélange d'au moins deux ou d'au moins trois types cellulaires choisis parmi les myofibroblastes, les fibroblastes, les myocytes, les cardiomyocytes et les cellules musculaires, par exemple un mélange de myofibroblastes et de fibroblastes.

Les cellules peuvent être associés à d'autre cellules contractiles ou non contractiles comme des cellules endothéliales. Globalement toutes les cellules présentent dans le coeur peuvent être associées.

Plus particulièrement, les cellules formant un tissu contractile peuvent être ou comprendre des myofibroblastes qui expriment le marqueur alphaSMA.

Le tissu contractile est essentiellement formé de cellules différenciées : généralement, au moins 80% des cellules qui le forment sont des cellules différenciées (qui peuvent par exemple exprimer le marqueur myofibroblastique alphaSMA). Pourtant, les cellules qui forment le tissu contractile expriment toujours certains marqueurs cellulaires qui sont usuellement observés sur les cellules souches (mésenchymateuses), et non sur des cellules différenciées (qui en l'occurrence peuvent par ailleurs exprimer le marqueur alphaSMA et/ou la myosine).

Par exemple, le tissu contractile peut comprendre des cellules qui sont positives pour un ou plusieurs des marqueurs CD105, CD54, CD73, CD105 et CD90, plus particulièrement pour un ou plusieurs des marqueurs CD105, CD54, CD73 et CD105.

Par exemple, le tissu contractile peut comprendre des cellules qui sont négatives pour un ou plusieurs des marqueurs CD45, HLA-DR, CD14, CD19, CD34 et CD106, plus particulièrement pour un ou plusieurs des marqueurs CD45 et HLA-DR.

Le tissu contractile peut notamment comprendre des cellules qui sont positives pour un ou plusieurs des marqueurs CD105, CD54, CD73 et CD105, et qui sont négatives pour un ou plusieurs des marqueurs CD45 et HLA-DR.

Le tissu contractile peut par exemple comprendre des cellules qui sont positives pour les marqueurs CD105, CD54, CD73 et CD105, et qui sont négatives pour les marqueurs CD45 et HLA-DR. Ainsi, le tissu contractile peut comprendre des cellules qui sont positives pour le marqueur alphaSMA (et optionnellement positives pour la myosine), positives pour les marqueurs CD105, CD54, CD73 et CD105, et négatives pour les marqueurs CD45 et HLA-DR.

Alternativement ou complémentairement, la différenciation des cellules souches, notamment des cellules souches mésenchymateuses, en cellules formant un tissu contractile peut être induite jusqu'à activation de la voie de signalisation Wnt canonique. L'activation de la voie de signalisation Wnt canonique peut causer l'accumulation de bêta-caténine dans le cytoplasme et sa translocation dans le noyau pour agir comme co-activateur transcriptionnel de facteurs de transcription.

Le tissu contractile a conservé une activité paracrine inductible (notamment une activité paracrine inductible favorisant l'immunosuppression de cellules T). Le fait de présenter une activité paracrine inductible, notamment une activité immunosuppressive paracrine inductible, est utile pour les applications médicales visées (contrôle et diminution des risques de rejets immunitaires).

L'induction de cette activité paracrine peut conduire à :
une activation ou stimulation de l'IDO *(inducible indoleamine* 2,3-dioxygenase) et/ou à
une augmentation de l'expression de certaines protéines, notamment de certaines métalloprotéinases, telles que MMP2, MMP9 et MMP14, et/ou à
une augmentation de l'expression d'une ou plusieurs des molécules choisies parmi
   TSG6 (*tumor necrosis factor Inducible gène 6),*
   VEGF (*Vascular endothelial growth factor*)*,*
   PDGF (*Platelet-Derived Growth Factor*),
   angiopoïétine, IL1 (*Interleukin-1*),
   IGF1 (*Insulin-Like Growth Factor-1*),
   FSL1 (synthetic lipoprotein Pam2CGDPKHPKSF), et
   PGE2 (Prostaglandine E2).

L'induction de l'activité paracrine (de cellules) dudit tissu contractile peut donc être réalisée jusqu'à (en une quantité et/ou durée suffisantes pour) obtention d'un ou plusieurs des éléments suivants :
activation ou stimulation de l'IDO,
expression, ou augmentation de l'expression d'au moins une métalloprotéinase (telle que MMP9 et/ou MMP2 et/ou MMP14),
plus particulièrement jusqu'à (en une quantité et/ou durée suffisantes pour) obtention d'un ou plusieurs des éléments suivants :
activation ou stimulation de l'IDO,
sécrétion, ou augmentation de la sécrétion, de MMP9,
sécrétion, ou augmentation de la sécrétion, de MMP2, et
expression (membranaire), ou augmentation de l'expression (membranaire), de MMP14, plus particulièrement au moins jusqu'à activation ou stimulation de l'IDO et/ou expression ou augmentation de l'expression d'une ou plusieurs métalloprotéinases, plus particulièrement au moins jusqu'à activation ou stimulation de l'IDO et/ou sécrétion ou augmentation de la sécrétion de MMP9 et/ou MMP2, plus particulièrement au moins jusqu'à sécrétion ou augmentation de la sécrétion de MMP9.

Ainsi, après induction de l'activité paracrine, le tissu contractile peut comprendre des cellules, qui expriment MMP9, MMP2 et MMP14, plus particulièrement qui sécrètent MMP9, MMP2, plus particulièrement qui sécrètent MMP9.

Il a en outre été constaté que l'activité paracrine inductible (ou induite) peut même être supérieure à celle qui serait observée avec les cellules souches indifférenciées. Par exemple, les cellules (différenciées) du tissu contractile de la demande (contenu dans le support solde poreux) peuvent présenter une sécrétion de métalloprotéinase(s), plus particulièrement de MMP9 et MMP2, plus particulièrement de MMP9, qui est supérieure à celle observée avec des cellules souches mésenchymateuses (en culture classique 2D). Cette caractéristique est favorable à l'angiogenèse, et est donc utile pour les applications médicales visées, plus particulièrement pour une thérapie régénérative d'un tissu myocardique dans un contexte d'infarctus chronique.

Ainsi, le tissu contractile comprend des cellules contractiles lesquelles comprennent des cellules qui expriment le marqueur alphaSMA et optionnellement la myosine, et qui, après induction par TGFbêta1, sécrètent au moins une métalloprotéinase choisie parmi MMP9 et MMP2 et MMP14.

Le support (poreux) à complaisance d'élasticité peut par exemple être un gel, hydrogel, une structure à mémoire de forme. Le support tridimensionnel peut être en phase liquide et une fois délivré après activation peut être converti en phase solide comme par exemple solution, pâte, un gel, une suspension colloïde, un plasma. Le support peut être un cryogel ou support injectable avec mémoire de forme. Les constituants du support peuvent être des agents intelligents capables de s'assembler, de se déformer et éventuellement de se réassembler. Il s'agit de matériaux ayant tendance à s'assembler à l'échelon moléculaire pour former des nano, micro ou macrostructures ou combinaisons.

Le support peut être des filaments, une éponge, matériaux avec ultrastructure filaire, ultrastructure alvéolaire d'éponge, analogues structuraux à forme d'interface, poudre, conduits, sphère, microsphère, film, micro ou nanofibrilles, membrane lipidique, fibres, matrices, patches, tissus, structure tissée, bêta ou alpha structures impliquées dans leur formation. Le support peut être composé par l'association des différents supports ou d'empilement d'un même support. Cela peut être des films multicouches obtenus par microfluidique par exemple. Le support peut être fabriqué par des technique de type « 3D printed ».

Le support (solide) (poreux) peut être un support tissé ou un support non tissé, plus particulièrement un gel, un hydrogel, une mousse, une éponge.

Le support peut être fabriqué à partir de monomère, polymère ou combinaison. Il peut s'agir de matériaux naturels ou pas. Pour les matériaux naturels ils peuvent être éventuellement produits par des techniques de biologie moléculaires.

Le support peut être totalement, partiellement ou pas biodégradable.

Le support peut être fabriqué à partir de polymères, monomères, naturels ou pas. Tous les polymères peuvent être utilisés, synthétiques ou naturels ou combinaison dans la mesure où le support obtenu répond aux propriétés de différenciation et de contractilité comme défini précédemment notamment en termes de complaisance d'élasticité. On préfèrera cependant les polymères naturels qui ont des molécules d'adhésions. Parmi les polymères naturels : le collagène. Le collagène est le composant naturel de la matrice extracellulaire dans les tissus et est donc particulièrement adapté.

Le support peut contenir ou être fabriqué avec un polymère naturel comme le collagène, la gélatine, un produit dérivé du collagène, fibrinogène, fibrine, soie en incluant la soie FIBROIN qui pourrait avoir un intérêt particulier du fait de molécule d'adhésion particulière, algue, alginate, protéoglycanes, glycoprotéines, glycoaminoglycanes, alginate, agarose, l'acide hyaluronique, l'agar, le chitosane, le fibrinogène/fibrine, le chitosan carboxyméthylé, octasulfate de sucrose, dextrane, cellulose, cellulose méthylée, sépharose, protéine imitée par le SEPHADEX (comme le latex) ou leur association. Les propriétés de ces polymères peuvent éventuellement être modifiées. Le support 3D peut provenir du regroupement de différents supports tissu micro ou nano structures, des filaments ou tubes, d'éponge, de poudre, de conduit, de sphère, de microsphère, de film, micro ou nanofibrilles, membrane, fibre, maille, matrice, patch, feuille de tissu, tissu décellularisé (dépourvu de cellules), tissu tricoté, ouatine ou combinaison.

L'échafaudage tridimensionnel contient du collagène qui est constitué de collagène (I, II, III, IV, V, VI, VII, XI et d'autres collagènes), ou de l'association de différentes espèces. Le terme « collagène » désigne également collagène insoluble, collagène soluble, atélocollagène préparé en enlevant des télopeptides sur les extrémités des molécules de collagène en utilisant une protéase autre que la collagénase. Il peut s'agir d'un collagène de synthèse. Cela peut être également de la gélatine de synthèse recombinante. L'échafaudage tridimensionnel de collagène peut également être un tissu normal d'origine autologue, homologue ou hétérologue. Ce tissu peut être du tissu décellularisé. Différents protocoles pour décellulariser les tissus peuvent être utilisés (par choc osmotique, détergents ioniques et non-ioniques, digestions protéolytique, traitements par DNase/RNase). La décellularisation peut être couplée à des techniques visant à modifier les propriétés du support comme la porosité. Le tissu décellularisé pourrait éventuellement être utilisé mais le problème de ces tissus est leur rigidité. En effet après extraction des cellules il y a une compaction des espaces vides et la rigidité augmente. Il s'agit également de tissus peu poreux. Les méthodes pour augmenter la porosité de ces tissus rendent ces tissus plus immunogènes et perdent des propriétés mécaniques de ces tissus.

Le collagène peut être purifié à partir de tissu contenant le collagène : autologue, homologue ou hétérologue comme uretère, péricarde, matrice tissulaire comme le coeur, sous muqueuse comme la sous muqueuse intestinale de porc « SIS », vaisseau sanguin, tendon, fascia, derme décellularisé ou pas, aponévrose, membrane type membrane amniotique, dure-mère. Ce pourrait être des copies synthétiques du collagène telles que des fibres de polymères ou des peptides formant des fibrilles. Le collagène peut être chimiquement modifié et le produit obtenu par succinylation ou estérification ou formation de carboxyamides, ou désamination des collagènes.

L'échafaudage peut être fabriqué à partir de polymères synthétiques organiques tels que poly (acide lactique) (PGA) et/ou poly (DL-lactide-Co-glycolide) (PLGA) et/ou poly (DL-lactide-Co-caprolactone) (PCL), un dérivé de collagène tel que la gélatine, un polypeptide obtenu par l'hydrolyse du collagène, collagène dénaturé par le chauffage. Des polymères synthétiques liés au collagène peuvent être choisis parmi Poly(3-hydroxybutyrate) (PHB)+++, poly(e-caprolactone) (PCL), soie, acide poly-lactique (PLA), polyamide (PA), l'acide polylactique (PLA), l'acide polyglycolique (PGA), poly (L-lactique) (PLLA), PLGA, poly (anhydrides) (PA), le polycarbonate (PC) les hydroxy acides, poly ortho- esters (POE), propylfumarates) (PPF), polysaccharides, la polylactone (PL), des poly caprolactones, polyamides, acides de polyamino, polyacétals des polyphosphazènes (PPZ), polycyanoacrylates biodégradables, les polyuréthanes biodégradables (unité centrale), polysaccharides, polypyrrole, polyanilines, polythiophène, polystyrène, polyester (PE), polyuréthanes non-biodégradables, polyurées, poly (éthylène-téréphtalate) (PET), poly (acétate de vinyle d'éthylène), polypropylène, polyméthacrylate, polyéthylène, polycarbonates, poly oxyde d'éthylène, poly alcool de vinyle (PVA), fuseau-tex (polytétrafluoroéthylène), dacron (téréphtalate de polyéthylène), polytétrafluoroéthylène (PTFE), poly-éthylène-glycol (PEG), polyglycérol sébacate (PGS), copolymères décrits ci-dessus, avec l'un des additifs ci-dessus, et mélanges de l'un des polymères, des copolymères, et des additifs entre eux et association de dérivés synthétiques avec les produits biologiques.

Préférentiellement on utilisera les collagènes purifiés de type I, Il et III. Certains collagènes ont des propriétés d'adhérence spécifique et pourront alors être associé comme collagène IV.

L'échafaudage peut être une matrice de type éponge obtenu par réticulation déshydratation hydrothermique (DHT). L'échafaudage peut être aussi une mousse, par une mousse de gélatine. Cela peut être une mousse de collagène comme GELFOAM^{™}. La matrice DHT correspond à des matrices obtenues par liaison croisée déshydrothermique. La fabrication de ces supports peut s'accompagner d'une étape de lyophilisation ou la température est abaissée en dessous de zéro degré pour augmenter la porosité du support puis une réticulation haute température basse pression est effectuée pour réticuler le support. Une matrice obtenue par DHT correspond aux matrices ULTRAFOAM^{™} de chez Bard. Une matrice fabriquée avec du collagène de type I et de type III peut être utilisée comme une éponge hémostatique. Le collagène initial est soluble dans une solution de 0,5% w/w de collagène dans acide acétique 0.05 M pH 3.5. La préparation est desséchée par le froid de manière contrôlée afin d'obtenir des éponges. La taille des pores et la distribution dépend principalement de la rapidité de la congélation (0,25-1°C/min.), de la température de congélation finale de (-90°C à -5°C). Cette éponge est alors soumise à une réticulation par DHT (105°C pendant 16h pour une pression inférieure à 100 mTorr) pour introduire des liaisons covalentes entre les chaines du collagène sans le dénaturer en gélatine. Pendant la fabrication le support peut être orienté en jouant par exemple sur le gradient de température.

Le collagène peut être associé à des glycoaminoglycanes. Les échafaudages peuvent éventuellement être fabriqués par une technique d'électrofilage *(electrospinning)* mais le problème de cette approche est que les supports ont une complaisance d'élasticité très élevée et il y a une difficulté à coloniser ces supports.

Le support peut être orienté. Les supports ont une porosité très importante proche de 98%. La porosité définie la capacité du support à laisser passer les liquides. Le support peut avoir une structure d'éponge à l'échelon ultra-structural. Cette structure d'éponge va favoriser la mise en cage initiale des cellules avec rétention dans le support. Certains supports se comportent également comme des éponges avec aspiration sous l'effet de la compression initiale des supports, et rétention à l'intérieur des cellules et des liquides. Dans le support il existe initialement une migration très importante des cellules qui vont permettre de coloniser rapidement l'intégralité du support dans la première semaine. Lorsque les structures contractiles se développent cette capacité de migration est perdue. Cette capacité de migration peut être éventuellement utilisée pour coloniser d'autres supports 3D mis au contact. Cette approche permet de réaliser des cultures à très faible coût dans un environnement 3D sans avoir à faire des cultures itératives dans des systèmes 2D. D'autre part, cette culture 3D permet de cultiver plus de cellules dans un espace limité.

Le support peut avoir des pores de taille intermédiaire pour permettre la diffusion des nutriments, la colonisation cellulaire *in vitro,* la colonisation cellulaire *in vivo.*

La porosité du support solide poreux est adaptée à la présence de cellules contractiles dans ces pores. La porosité peut notamment être de 20 à 200 µm, par exemple de 50 à 100 µm. Cette porosité peut être homogène ou non-homogène.

Le support peut être orienté. Les supports orientés à complaisance d'élasticité favorisent la différenciation vers des cellules contractiles. Les forces développées dans des structures orientées sont supérieures. Il existe une synergie d'action entre l'orientation des supports et une éventuelle stimulation mécanique de ces supports.

Le support peut être réticulé. Il existe différentes méthodes de réticulation chimique (comme par exemple aldéhydes, isocyanates, carbodiimides), physique, biologique et combinaison. Dans les réticulations physiques par exemple l'UV, déshydrothermale (DHT). Pour les réticulations chimiques on pourra choisir des réticulants de préférence non toxiques pour les cellules comme diphenylphosphorylazide, carbodiimides, EDC (1-ethyl-3-(3-dimethylaminopropyl-carbodiimide hydrochloride), EDC/NHS EDC (1-ethyl-3-(3-dimethylaminopropyl-carbodiimide hydrochloride) en présence de NHS (N-hydroxy-succinimide), EGS (ethylene glycol-bis (succinic acid N-hydroxysuccinimide ester), génipine. Eventuellement le glutaraldéhyde peut être utilisé s'il est détoxifié. Il peut être possible d'utiliser l'acide citrique comme agent de réticulation lors de procédé d'electrofilage (« *electrospinning* ») du collagène par exemple. On peut également utiliser une réticulation enzymatique comme par exemple la transglutaminasase. La réticulation peut avoir lieu *in vivo.* Le support peut être réticulé plusieurs fois *in vitro* et *in vivo.* La réticulation chimique fait diminuer les sites d'adhésions pour les cellules. Nous proposons d'associer la réticulation à des molécules d'adhésions pour améliorer la fonctionnalité des supports. Nous avons observé qu'il était possible de réticuler le support par la génipine (concentration <10 mM pendant 24h à 37°C, concentration optimale 1,5 mMol pendant 24h à 37°C) sans modifier ni la capacité des cellules à se différentier en cellules contractiles dans le support, ni la préservation de la contractilité du support et la capacité paracrine inductible des cellules. Les agents réticulants peuvent influer sur la capacité de différenciation des cellules. Cela est très intéressant car normalement les supports réticulés ont une rigidité supérieure et donc une moindre contractilité. Nous avons observé de la contractilité dans les supports réticulés DHT réticulés par la génipine ou réticulé par EDC/NHS. Les cellules se différencient en myofibroblastes et l'activité paracrine inductible est la même dans les supports réticulés ou pas. L'activité contractile reste présente dans les supports réticulés.

La structure du support solide poreux peut se présenter sous la forme d'un échafaudage tridimensionnel. Elle peut par exemple comprendre un polymère (tels que ceux listés ci-dessus) sous forme réticulée. La réticulation du polymère (par exemple du collagène, notamment du collagène de type I ou de type III) peut par exemple être faite par voie chimique, plus particulièrement par génipine (par exemple de la génipine à 0.1-10 mM) ou par 1-ethyl-3-(3-dimethylamino propyl) carbodiimide (EDC) et/ou par voie physique, notamment par réticulation déshydratation hydrothermique (DHT).

Le support à complaisance d'élasticité peut en outre comprendre des molécules d'adhésion, notamment des molécules de peptide(s) d'adhésion, plus particulièrement des molécules de peptide RGD (Arg-Gly-Asp). Les molécules d'adhésion incluent des poly nucléotides, des peptides d'adhésion qui incluent peptides, polypeptides, protéines ou molécules capables de se lier aux récepteurs cellulaires favorisant l'adhérence cellulaire avec de hautes affinités comme c'est le cas des récepteurs intégriniques et selon les données bibliographiques actuelles. Les peptides d'adhésion peuvent être formés d'acides aminés naturels ou non et/ou analogues. Un ou plusieurs acides aminés du peptide d'adhésion peut être substitué. Les peptides d'adhésion incluent les peptides, polypeptides ou protéines ou molécules (d'origines naturelles ou pas) contenant la séquence d'adhésion initiale. Il peut s'agir également de structure peptidomimétique pouvant jouer la même fonction que le peptide. Les termes polypeptide, peptide et protéine peuvent être employés l'un pour l'autre. Ceci comprend aussi l'addition d'une entité chimique telle qu'un groupe d'hydrate de carbone, un groupe phosphate, un groupe farnésyle, un groupe isofarnésyle, un groupe acide gras, un promoteur de liaison pour la conjugaison, fonctionnalisation, ou toute autre modification ou association organique ou inorganique de molécule y compris le polyéthylène glycol (PEG) ou toute autre molécule synthétique. Ces modifications peuvent également inclure la cyclisation du peptide contenant la séquence d'adhésion. Plusieurs peptides d'adhésion peuvent être associés et le peptide peut se rapporter à un peptide individuel ou à une collection de peptides Les molécules ou les peptides d'adhésion peuvent être modifiés et appartenir aussi à une même molécule ou à une molécule servant d'intermédiaire pour la liaison au support.

Sont en outre inclus dans le peptide, les peptides d'adhésion qui visent à interagir avec le récepteur d'adhésion lui-même, avec les co-récepteurs (impliqués non nécessairement dans l'adhésion). La liaison à ces co-récepteurs modifie et/ou l'adhésion et/ou l'activation et/ou la transduction du récepteur.

Les intégrines reconnaissant le collagène naturel sont les intégrines alpha1, alpha2, alpha10 et alpha11 associée à la chaine bêta1.

Tel que décrit dans la présente demande, le peptide d'adhésion pourrait être le peptide RGD correspondant à l'arginine (R) - la glycine (G) -l'acide aspartique (D), motif de reconnaissance de l'intégrine sur la fibronectine décrite par Pierschbacher *et al.* (« RGD linéaire » Pierschbacher MD et al., Nature. 1984, May 3-9;309(5963):30-3), ou sur la vitronectine comme décrite par Plaff M. *et al.* (« RGD cyclique » Plaff M et al., J Biol Chem. 1994 Aug 12;269(32):20233-8). Le « motif RGD » inclut également tous les ligands peptidiques qui interagiront avec l'un des récepteurs intégriniques, tous les récepteurs alphav (5 récepteurs) : αᵥβ1, αᵥβ3, αᵥβ5, αᵥβ6, αᵥβ8, αII_{b}β3, α₅β1, α8β1. D'autres peptides d'adhésion tels que les peptides PHSRN ou domaine obligatoire protéoglycane de la matrice extracellulaire (domaines obligatoires d'héparine) basés sur X-B-B-X-B-X (SEQ ID NO : 5) ou X-B-B-B-X-X-B-X (SEQ ID NO : 6), modèles d'ordre de B-B-X-B (SEQ ID NO : 7), où B est un aminoacide de base et X est un hydroxy aminoacide, YIGSR (SEQ ID NO : 8) (Iwamoto et al., Science. 1987 Nov 20;238(4830):1132-4) et IKVAV (SEQ ID NO : 9) (-Ile-Lys-Val-Ala-Val-) (Tashiro et al., J Biol Chem. 1989 Sep 25;264(27):16174-82) RYWLPR (SEQ ID NO : 10) ou RNIAEIIKDI (SEQ ID NO : 11) (Liesi P et al., FEBS Letters. Volume 244, Issue 1, 13 February 1989, Pages 141-148) de la laminine, REDV (SEQ ID NO: 12) (Massia SP et al., J Biol Chem. 1992 Jul 15;267(20):14019), PHSRN (SEQ ID NO : 13) (-Pro-His-Ser-Arg-Asn) (Aota S et al., J Biol Chem. 1994 Oct 7;269(40):24756-61) ou KNEED (SEQ ID NO : 14) (Altroff H et al., J Biol Chem. 2001 Oct 19;276(42):38885-92 ; Wong JY et al., Biomaterials. 2002 Sep;23(18):3865-70) ou EILDV (SEQ ID NO : 15) de la fibronectine, domaine obligatoire protéoglycane des protéines d'adhésion telles que KRSR (SEQ ID NO : 16) (Dee KC et al., J Biomed Mater Res. 1998 Jun 5;40(3):371-7 ; Rezania A et al., Biotechnol Prog. 1999 Jan-Feb;15(1):19-32) ou FHRRIKA (SEQ ID NO: 17) (Rezania A et al., Biotechnol Prog. 1999 Jan-Feb;15(1):19-32), séquence de VAPG (SEQ ID NO : 18) et de KQAGDV (SEQ ID NO : 19) de l'élastine (Mann & West et al., J Biomed Mater Res. 2002 Apr;60(1):86-93), GFOGER du type I de collagène (Emsley J et al., Cell. 2000 Mar 31;101(1):47-56), DEGA (Rabenau KE et al., Oncogene. 2004 Jun 24;23(29):5056-67).

Dans un mode de réalisation, le support est modifié avec une molécule correspondant à la fibronectine notamment à la région contenant le motif RGD. Au niveau de la fibronectine l'expression du RGD est linéaire alors que dans la vitronectine il s'agit de la forme cyclique. Les séquences peuvent par exemple être RGD, RGDS (SEQ ID NO : 20), GRGD (SEQ ID NO : 21), RGDV (SEQ ID NO : 22), RGDT (SEQ ID NO : 23), GRGDG (SEQ ID NO : 24), GRGDS (SEQ ID NO : 1), GRGDY (SEQ ID NO : 25), GRGDF (SEQ ID NO : 26), YRGDS (SEQ ID NO : 27), YRGDDG (SEQ ID NO : 28), GRGDSP (SEQ ID NO : 29), GRGDSG (SEQ ID NO : 30), GRGDSY (SEQ ID NO : 31), GRGDVY (SEQ ID NO : 32), GRGDSPK (SEQ ID NO : 33), CGRGDSPK (SEQ ID NO : 34), CGRGDSPK (SEQ ID NO : 35), CGRGDSY (SEQ ID NO: 36), cyclo(RGDfK) (SEQ ID NO: 37), YAVTGRGD (SEQ ID NO: 38), AcCGGNGEPRGDYRAY-NH2 (SEQ ID NO : 39), AcGCGYGRGDSPG (SEQ ID NO : 40) et RGDPASSKP (SEQ ID NO : 41), les variants cycliques de ces peptides, les variants multivalents linéaires ou ramifiés (Dettin M et al., 2002, J. Biomed. Mater. res. 60:466-471 ; Monaghan S et al., 2001, Arkivoc, 2:U42-49 ; Thumshirn G et al. 2003, Chemistry 9:2717-25 ; Scott ES et al., 2001, J.Gene Med. 3:125-134), ainsi que les combinaisons de deux ou plus de deux de ces peptides.

Pour être efficace le peptide d'adhésion, s'il n'est pas inclus dans la structure primaire du polymère nature, il doit être fixé au support. Si le motif d'adhésion n'est pas fixé de manière covalente, il a un effet inverse sur les cellules (pas de survie, diminution de la contractilité). Le motif d'adhésion sert de pont entre le support et l'appareil contractile de cellules et il doit nécessairement être fixé. Des molécules de peptide(s) d'adhésion, plus particulièrement des molécules de peptide RGD, peuvent être liées à la structure (polymérique) du support solide (poreux) par une liaison covalente, optionnellement *via* un bras espaceur, plus particulièrement un bras espaceur de 30 à 40 Angström.

Le support solide (poreux) peut être complètement, partiellement ou non biodégradable.

Le support solide (et le biomatériau qui le contient) ne comprend pas d'extraits tumoraux (de type MATRIGEL^{®}), plus particulièrement d'extrait protéique d'une tumeur de Engelbreth-Holm-Swarm (EHS).

Le procédé tel que décrit dans la demande comprend un support de contractilité qui est un échafaudage tridimensionnel de polymère réticulé, plus particulièrement de collagène réticulé, qui présente une porosité de 20 à 200 µm, et qui optionnellement comprend des molécules de peptide d'adhésion, plus particulièrement des molécules de peptide RGD, liées de manière covalente à la structure polymérique du support solide.

Le support peut être associé à un agent, notamment un agent de couplage.

L'agent peut être associé sous forme libre, absorbé, adsorbé ou fixé. La fixation peut être réversible ou association. L'agent peut être sécrété par des cellules, ou le produit de ces cellules y compris des cellules génétiquement modifiées, ou produit par des cellules soumises à différentes conditions de stress (hypoxique, chimique, physique ou autre) ou environnementale. L'agent peut être présent dans le support *in vitro, in vivo* avant, pendant ou après l'implantation du support. L'agent peut éventuellement être couplé au support. La fixation peut être réversible. L'agent peut être relargué par le support. La dégradation du support peut permettre le contrôle du relargage de l'agent. L'agent peut être présent sous la forme de sécrétome ou d'exososomes.

Les agents peuvent être de manière non limitative des protéines, oligoprotéines, peptides naturels ou pas, des DNA, microRNA, mRNAs, mitochondries. Ils peuvent être des facteurs de croissance, des cytokines, chimiokines, hormones, médicaments par exemple. Il peut s'agir également de microvésicules ou de structure type exosomes. Ces exosomes peuvent être obtenus éventuellement à partir de plusieurs types de cellules placées dans des conditions visant à obtenir des propriétés particulières de ces exosomes. Les exosomes produit par un type cellulaire peuvent par exemple servir à « *primer »* un autre type cellulaire qui lui sera transplanté. Par exemple, les exosomes dérivés des cardiosphères peuvent servir à activer en *primant* un fibroblaste qui lui sera transplanté *in vivo* et non la cellule à l'origine des exosomes.

Les agents de couplage peuvent avoir un impact fonctionnel sur les cellules du support ou le support lui-même ou un impact fonctionnel sur les cellules myocardiques ou la matrice extracellulaire cardiaque, recrutement cellulaire et fonction de cellules notamment dans le cadre de l'infarctus (aigue, subaigüe chronique), cardiopathie ischémique, ischémie coronarienne, cardiopathie non ischémique, myocardite, insuffisance cardiaque, choc cardiogénique, conditions myocardiques.

Le support peut être par exemple associé à des agents favorisant l'angiogénèse, anti-arythmique, la contractilité, contrôlant le remodelage ventriculaire, la taille de l'infarctus, effet sur l'adhésion des cellules, la survie, la prolifération, la régénération, la signalisation (*signaling*), l'apoptose, la migration, le *homing,* la différenciation, connexion entre les cellules et cellules entre elles, contractilité, le recrutement des cellules, le contrôle des réactions inflammatoires et immunitaires. L'agent peut être un agent immunosuppressif ou régénératif. Les agents immuno-suppressifs peuvent favoriser la régénération *in vivo.* L'agent peut favoriser une réparation cardiaque en plusieurs temps, en facilitant par exemple l'utilisation d'une thérapie cellulaire ultérieure ou séquentielle ou répétitive, ou plusieurs types de cellules peuvent être employés. L'agent peut par exemple préserver le tissu pour une thérapie cellulaire ultérieure. Les différents agents et effets peuvent être associés.

Il peut s'agir d'agent favorisant l'adhérence cellulaire médiée par les sélectines avec par exemple modification du support de contractilité par modification avec des sucres.

L'agent peut être éventuellement délivré dans le support ou dans la zone thérapeutique cible de la fonctionnalisé par le biais d'un dispositif additionnel comme par exemple un filet de contention ventriculaire, un dispositif visant à protéger ou à maintenir le support de contention. Le facteur peut être délivré en même temps ou secondairement par voie endovasculaire injection endocoronaire, endoveineuse, systémique, injection intramyocardique (endoventricule, épicardique), injection dans le péricarde ou libération dans le péricarde par des dispositifs pas directement au contact du coeur mais destiné à le traiter. L'agent peut être également produit par des préparations situées dans des régions ectopique non cardiaques puis transféré ou non dans la région du coeur comme des lambeaux (*flaps*) vascularisés ou non vascularisés, ou juste une région d'intérêt transférée au niveau du coeur en libre. Sont dans le domaine l'utilisation des *flaps* épiploïques par exemple ou des supports préparés dans la cavité péritonéale et ensuite appliqués sur le coeur ou cavité péricardique.

Les agents en général y compris les cellules décrites dans la demande peuvent être modifiées génétiquement en utilisant des modes de modification génétiques virales ou non virales.

Dans ces agents sont inclus des facteurs impliqués dans la différenciation des cellules souches vers des cellules contractiles, la transformation de fibroblastes vers des myofibroblastes, facteurs impliqués dans l'induction de l'activité paracrine des MSC, pouvant les modifier ou prolonger leur sécrétion ou des agents permettant de contrôler la colonisation cellulaire des supports y compris la réaction inflammatoire.

Parmi les agents que l'on peut associer au support il y a l'interféron Gamma, le TGFbêta1, facteurs libérés les MSC par exemple, facteur qui vont augmenter la voie de signalisation Wnt (*Wnt signaling*) surtout la forme cathionique Wnt/bêtacathénine dont le Wnt3a.

Le biomatériau peut être soumis à un stimulus physique.

Le support peut être stimulé physiquement en étant soumis à un stimulus mécanique comme par exemple électrique, mécanique élongation, compression extension, accrochés à un ressort ou fixé (stress isométrique), électromagnétique, thermique. Le support peut être électrostimulé. L'électrostimulation joue un rôle dans la différentions vers des cellules contractiles de type cardiomyocytes. L'électrostimulation jour également un rôle dans la différenciation des fibroblastes vers des myofibroblastes. La stimulation mécanique diminue la différenciation des MSC vers des cellules adipocytaire en inhibant les voies SMAD (« *SMAD pathways* »).

Le procédé tel que décrit dans la présente demande présente un support de contractilité qui est
un solide, un gel ou un hydrogel,
optionnellement réticulé et/ou optionnellement poreux,
pouvant avoir la structure d'une éponge ou d'une mousse ;
et un support qui peut être associé à un agent et/ou peut être stimulé physiquement.

Le support peut être développé dans un bioréacteur.

Le support cellularisé tel que décrit peut être mis dans un bioréacteur. Ces bioréacteurs peuvent avec un stimulus mécanique, qui peut être produit par le flux liquidien par exemple, stimulé physiquement ledit support cellularisé. La demande telle que décrite est ainsi relative à une (nouvelle) méthode de production de biomatériaux en bioréacteurs associant différents types de cellules et différents stimuli. Les stimuli électriques et mécaniques peuvent augmenter la différenciation vers des cellules contractiles et l'activité paracrine des cellules associées.

Le biomatériau contractile tel que décrit est spécialement adapté à la thérapie régénérative du tissu myocardique, notamment du tissu myocardique humain. Il est particulièrement destiné à des sujets dont le tissu myocardique est ischémique et inflammatoire, par exemple un tissu myocardique qui a souffert d'un infarctus aigu ou chronique.

Le biomatériau contractile tel que décrit peut notamment être destiné à être administré par voie péricardique (lors de pontage coronaire, ou lors de mise en place d'une assistance mécanique cardiaque), par sternotomie, par voie mini-thoracotomie, par voie xiphoïdienne, ou par péricardotomie et/ou par voie mini-invasive.

Le support solide (poreux) (et donc le biomatériau contractile) tel que décrit peut être de toute forme et dimensions que la personne du métier jugera appropriées, notamment en regard de l'application médicale visée (par exemple en regard de la taille de la zone myocardique visée), et en regard de la voie d'administration envisagée.

Il peut par exemple être sous forme ronde, ovale, ovoïde, et/ou avoir une surface de 0,5 à 10 cm², et/ou avec une épaisseur de 0,1 mm à 1 cm.

Le biomatériau tel que décrit dans la demande est contractile.

Le biomatériau contractile tel que décrit dans la demande présente une complaisance d'élasticité de 0.1 à 30 kPa⁻¹, plus particulièrement de 0.1 à 3 kPa⁻¹ pour les myofibrobastes et plus particulièrement de 5 à 15 kPa⁻¹ pour les autres cellules contractiles.

Les propriétés de contraction et de relaxation du biomatériau de la demande sont adaptées à une application sur un tissu myocardique *in vivo.* Ces propriétés de contraction et de relaxation peuvent être observées par application d'un stimulus de contraction entraînant la réduction d'une dimension du biomatériau, et par arrêt de l'application de ce stimulus. Ce stimulus de contraction peut par exemple être par une stimulation électrique en tétanus (par exemple appliqué avec du courant alternatif) ou par stimulation chimique au KCI.

La relaxation du biomatériau cellulaire peut être de même amplitude que la contraction qui l'a précédée.

Le biomatériau cellulaire répond à une courbe de Hill, c'est-à-dire qu'il présente une vélocité de contraction qui est en relation hyperbolique avec la tension qu'on lui applique.

Les cinétiques de contraction et de relaxation du biomatériau cellulaire sont proches de ou sensiblement non différentes de celles du placenta humain.

Le biomatériau cellulaire de la demande peut par ailleurs présenter des propriétés d'élongation élastique : il peut par exemple être soumis à une élongation mécanique de l'ordre de 10% à 20%.

De manière remarquable, le biomatériau cellulaire contractile peut être conservé sans être congelé : une simple hypothermie suffit.

En effet, le biomatériau cellulaire conserve des propriétés de contractilité après avoir été conservé en solution isotonique pendant 5 jours (notamment pendant 5 à 10 jours) à une température supérieure à 0°C et inférieure à 10°C (notamment une température supérieure à 0°C et inférieure à 4°C).

Le biomatériau cellulaire contractile tel que décrit peut même conserver une activité paracrine inductible après avoir été conservé en solution isotonique pendant 5 jours (notamment pendant 5 à 10 jours) à une température supérieure à 0°C et inférieure à 10°C (notamment une température supérieure à 0°C et inférieure à 4°C).

Le biomatériau cellulaire contractile tel que décrit peut donc être conservé simplement au réfrigérateur. Le biomatériau cellulaire peut par exemple être conservé dans une solution isotonique, du sérum ou du plasma, ou un milieu de culture (cellulaire). Il peut par exemple être contenu dans une plaque de culture cellulaire, telle qu'une plaque à 96 puits, ou dans un tube de culture cellulaire (tel qu'un tube cylindrique de 50 mL).

En outre, le biomatériau cellulaire tel que décrit est immédiatement utilisable à l'issue de la conservation en conditions hypothermiques.

La demande telle que décrite se rapporte plus particulièrement au biomatériau cellulaire contractile tel queci-dessous décrit et ci-dessus illustré et à ses applications médicales.

Un biomatériau cellulaire contractile susceptible d'être obtenu par le procédé tel que décrit est adapté à la thérapie régénérative du tissu myocardique et comprend :
- un support solide optionnellement poreux qui présente une complaisance d'élasticité de 1 à 30 kPa⁻¹ (complaisance avec ou sans cellule, plus particulièrement sans cellule), et
- un tissu cellulaire contractile qui est contenu dans le support solide optionnellement poreux,
les cellules contractiles comprenant des cellules choisies parmi les myofibroblastes, les fibroblastes, les myocytes, les cardiomyocytes et les cellules musculaires et les progéniteurs de ces cellules.

Il peut en outre présenter une activité paracrine inductible par IFNgamma, l'activité paracrine inductible comprenant une activité d'immunosuppression de cellules T.

Le biomatériau tel que décrit peut en outre comprendre une colle, telle qu'une colle de fibrine, à sa périphérie, cette colle étant destinée à favoriser la fixation du biomatériau sur le tissu myocardique. Cette colle est néanmoins optionnelle dans la mesure où le matériau peut présenter une adhésivité spontanée sur le tissu myocardique.

Le biomatériau tel que décrit peut être conservé à une température supérieure ou égale à 0°C, plus particulièrement à une température supérieure à 0°C, plus particulièrement à une température supérieure à 0°C et inférieure à 32°C, plus particulièrement à une température supérieure à 0°C et inférieure à 10°C, notamment à une température supérieure à 0°C et inférieure à 4°C.

Le biomatériau tel que décrit peut être conservé au-delà de 24 heures, par exemple pendant 5 jours ou plus de 5 jours (notamment jusqu'à 10 jours).

Le biomatériau tel que décrit peut être conservé dans tout type de contenant que la personne du métier considère approprié, par exemple dans le puits d'une plaque à 96 ou 24 puits, ou un tube de culture cellulaire (par exemple un tube de 50 mL de culture cellulaire).

Le biomatériau tel que décrit peut être conservé dans un milieu tel qu'un milieu de culture cellulaire. Le milieu dans lequel (ou la solution dans laquelle) le biomatériau est conservé ou placé avant administration n'a pas à être un milieu (ou une solution) hypothermique.

Le biomatériau tel que décrit peut permettre de contrôler la migration cellulaire *in vitro* et *in vivo.* Le biomatériau tel que décrit peut permettre une thérapie cellulaire ciblée en préservant les cellules dans le support *in vivo* et en préservant leur phénotype immunosuppressif pro-régénératif.

Le biomatériau tel que décrit dans la demande est notamment destiné à être utilisé dans le traitement d'un infarctus aigu (0-7 jours), subaigu (7 jours à 1 mois) ou chronique (après 1 mois) afin de limiter la taille de l'infarctus, préserver le tissu myocardique, contrôler le remodelage ventriculaire, favoriser l'angiogenèse, favoriser la régénération cardiaque. Il peut être utilisé pour le traitement d'une insuffisance myocardique, notamment d'une insuffisance myocardique ischémique ou post-infarctus (par cicatrisation du tissu myocardique infarci et/ou restauration d'une contractilité du tissu myocardique infarci). Le biomatériau avec la sécrétion de très nombreux agent immunosuppresseur et anti-inflammatoire peut être également intéressant dans les myocardites. Il peut être également intéressant dans les pathologies cardiaques inflammatoires comme par exemple maladies rhumatismales.

Le biomatériau tel que décrit dans la demande est notamment destiné à être utilisé dans le traitement d'une cardiomyopathie dilatée ou hypertrophique, d'une myocardite, d'un vieillissement pathologique (ou normal) du coeur, d'une fibrose myocardique, d'un rejet de greffon cardiaque, d'une maladie infectieuse du coeur (par exemple la maladie de Chaga).

Le biomatériau tel que décrit dans la demande peut être utilisé dans le traitement régénératif (ou cicatriciel) d'un tissu cardiaque, d'un tissu musculaire ou d'un tissu humain non contractile, plus particulièrement dans le traitement d'une insuffisance myocardique post-infartus. Il peut notamment restaurer une contractilité du tissu myocardique.

Le biomatériau tel que décrit peut également être implanté au contact ou dans du tissu musculaire contractile, pas nécessairement cardiaque, comme du tissu musculaire pour délivrer des agents visant à traiter les muscles ou l'individus en général.

Le biomatériau tel que décrit peut également permettre de délivrer *in vivo* une thérapie ciblée après application au contact de tissue pathologique pour favoriser leur régénération. Le support permet de préserver les cellules à distance de la réaction inflammatoire et de préserver le phénotype immunosuppressif pro régénératif *in vivo.* L'engagement des cellules dans des structures contractiles va prévenir leur migration *in vivo.* On aura donc un maintien des cellules dans le site de délivrance et ainsi la possibilité de délivrer une thérapie ciblée. Possible application par exemple sur le foie.

L'approche que nous avons pour le stockage, le transfert et la délivrance de MSC multipotentes est une approche qui ne nécessite pas la congélation des cellules au moins pendant la phase finale. L'approche consiste donc à ne pas utiliser les MSC multipotentes mais à les différencier dans un support 3D avec les facteurs appropriés. Ce support va leur permettre d'interagir entre elles et entre elles et le support de telle sorte que ces cellules vont pouvoir être organisées en structure tissulaire comme du tissu osseux, cartilagineux, adipeux par exemples. Dans ces structures les MSC pourront être transférées en hypothermie >0°C, de préférence entre 0-10°C.

### EXEMPLES

### Exemple 1 : production d'un biomatériau contractile

### Préparation du support solide poreux

Quatre types de matrices ont été produites :
- une matrice de collagène réticulée par déshydratation hydrothermique (DHT),
- une matrice de collagène réticulée par DHT et comprenant des molécules de peptide d'adhésion, à savoir des molécules de RGD (Arg-Gly-Asp),
- une matrice de collagène réticulée par génipine,
- une matrice de collagène réticulée par génipine et comprenant des molécules de peptide RGD.

La matrice de collagène réticulée par DHT a été produite. La matrice obtenue par DHT correspond aux matrices Ultrafoam ^{™} de chez Bard. La matrice a été fabriquée avec du collagène de type I et de type III et utilisée comme une éponge hémostatique. Le collagène initial est soluble dans une solution de 0,5% w/w de collagène dans l'acide acétique 0,05 M pH 3,5. La préparation est desséchée par le froid de manière contrôlée afin d'obtenir des éponges. La taille des pores et la distribution dépend principalement de la rapidité de la congélation (0,25-1°C/min), de la température de congélation finale de (-90°C à -5°C). Cette éponge est alors soumise à une réticulation par DHT (105°C pendant 16h pour une pression inférieure à 100 mTorr) pour introduire des liaisons covalentes entre les chaines du collagène sans le dénaturer en gélatine. Les fibres de collagène ont spontanément tendance à se grouper en structure poreuse. Cette capacité reste présente avec de la gélatine mais la porosité est plus faible. Pendant la congélation il est possible de faire un gradient thermique pour orienter le support.

La matrice peut également être produite sous forme de mousse poreuse de gélatine (« *foam templating* »).

Une phase gazeuse (azote) est générée par réaction entre l'acide sulfamique et le nitrite de sodium dans une solution de gélatine et en présence d'un polymère tensioactif et de SDS. La mousse est préparée à 45°C puis placée à 5°C pour prise en gel. Après purification, le gel physique est auto-réticulé avec de l'EDC et peut être lyophilisé. Les vides et interconnections sont de l'ordre de 230 et 90 microns, respectivement.

La matrice de collagène réticulée par génipine a été produite. Nous avons observé qu'il était possible de réticuler le support par la génipine (concentration <10 mM pendant 24h à 37°C, concentration optimale 1,5 mMol pendant 24h à 37°C). La génipine peut être utilisée pour des concentrations entre 0,1 et 10 mM. Pour des concentrations supérieures, la génipine est toxique pour les cellules. La réticulation peut se faire à température de la pièce ou à 37°C. La réticulation du support s'accompagne d'une réticulation plus ou moins importante du support. On peut réticuler jusqu'à 3 jours mais des tissus peuvent être réticulés pendant 1 mois. Plus la température augmente plus la réticulation est rapide.

Pour la fixation covalente du motif RGD nous avons utilisé un lieur (« *crosslinker* ») hétérobifunctionnel, le sulfo-LC-SPDP, qui permet de coupler un site NH2 du peptide avec un site amine présent sur le collagène. En modifiant à la fois le collagène et le peptide nous avons été capable de fixer la molécule de manière unidirectionnelle avec introduction d'un bras espaceur de 36 Angström. Durée de la fixation 48h. Durant la fixation des groupements thiols sont libérés dans le milieu et nous permettent de connaitre à la fois le nombre de site NH2 disponibles sur le collagène et également le nombre de molécules RGD fixés sur le support.

L'espaceur optimal est aux alentours de 36 Angström (40-50 Angström). Avec un effet progressif de zéro Angström à 35. De manière préférable le lieur peut être flexible et hydrophile. Dans un mode de réalisation, le lieur peut être fait d'acide aminé uniquement sans participation de l'agent utilisé pour la liaison croisée (« *crosslinking* ») dans l'espaceur. Des acides aminés de type glycine peuvent être utilisés. L'espaceur pour chaque glycine est d'environ 3,4 Angström. On peut alors avoir des oligopeptides qui contienne le motif GRGDS (SEQ ID NO : 1) et l'espaceur -GSGGGGGGS-GRGDS (SEQ ID NO : 2) ou -GGGGGGGGG-GRGDS (SQ ID NO : 3). La présence d'un acide aminé S améliore la présentation du peptide au niveau de la membrane. L'oligopeptide contenant le RGD sera alors fixé au -NH2 du collagène par une réaction classique chimique de type Azide ou isocyanate. On peut également utiliser des molécules de type ---CGSGGGGGGGS-GRGDS ou CGSGGGGGGGS-GRGDS et utiliser le Succinimidyl iodoacetate C6H6INO4 (SIA) (zéro lieur pour le couplage du peptide au collagène). L'idée est d'avoir un espaceur de 36 Angström fait principalement de glycine. On peut avoir un bras espaceur dont une partie est un polymère synthétique comme fait de HOOC-H2CH2C-PEH8-NH2 et utiliser le SIA pour fixer l'oligopeptide C-GRGDS par un groupement -SH et le EDC/NHS ou EDC ou le Sulfo-NHC pour lier le groupement carboxilique au groupement -NH2 du collagène. En utilisant le Sulfo-LC-SPDP on peut limiter le coût en utilisant un peptide modifié contenant un groupement -SH comme une cystéine de type -C-GGGG-GRGDS (SQ ID NO : 4). On a donc une cystéine avec groupement thiol sur lequel on peut faire une fixation sur le collagène en utilisant le Sulfo-LC-SPDP. La fixation finale donnera un bras espaceur de 36 Angström.

Pour les matrices comprenant du peptide G-RGDS, le peptide « RGD » a été utilisé à raison de 1 mg pour 10 mg de collagène. Les molécules de peptide RGD ont été fixées de manière covalente aux molécules de collagène du support par fonctionnalisation en phase hétérogène solide, en utilisant le sulfosuccinimidyl 6-(3'-(2-pyridyldithio)propionamido)hexanoate (sulfo-LC-SPDP).

La réticulation par la génipine n'a pas modifié la fixation des molécules d'adhésions. Cependant in *vivo,* le contrôle de la résorption de la matrice chez le rat immunocompétent est supérieur si les matrices sont réticulées avant la modification avec les molécules d'adhésions.

Dans chaque matrice produite, la taille des pores était comprise entre 20 et 200 µm.

Chaque matrice produite a été découpée en forme de disques de 6 mm de diamètre et de 3 mm d'épaisseur. Lorsqu'elles sont immergées dans le milieu de culture l'épaisseur des matrices diminue de 20%. La coupe des matrices est peu importante car les cellules ont la capacité de migrer dans la matrice. Eventuellement pour améliorer l'ensemencement initial si les concentrations de cellules sont très importantes une courte centrifugation peut être employée. L'épaisseur du myocarde humain est de 10 mm.

Chaque disque peut individuellement servir de support solide poreux.

Isolement de cellules souches mésenchymateuses.

Des cellules souches mésenchymateuses (*Mesenchymal Stem Cells* ou MSC) ont été isolées à partir de la moelle hématopoïétique présente dans les têtes de fémurs humains. Ces têtes de fémur provenaient de tissus qui avaient été sacrifiés lors du remplacement orthopédique de hanche par prothèse. L'isolement des cellules a été réalisé sur la fraction mononucléaire des cellules de cette moelle. La crête iliaque a été aspirée à partir de la tête de fémur réséquée. Chaque aspirat (3-30 mL) a été collecté dans un tube EDTA. Les aspirats ont été dilués à 1:1 dans une solution de HBSS *(Hank's Balanced Slat Solution).* Les cellules mononuclées ont été collectées par centrifugation avec un gradient de densité de 1000 G pendant 20 min à travers des tubes contenant UNI-SEP Maxi (polysucrose-sodium metrizoate). La fraction mononuclée est collectée et lavée sur une solution de HBSS. Les cellules ont été colorées au bleu de trypan pour identifier et collecter les cellules vivantes. Les cellules ainsi colletées ont été cultivées sur un milieu d'expansion dans des flacons standard de culture cellulaire (NUNC^{®} 75 cm²) à raison de 250 000 - 300 000 cellules par flacon. Les cultures ont été incubées à 37°C sous 5% de CO₂.

Le milieu d'expansion était un milieu de DMEM *(Dulbecco's Modified Eagle Medium* à faible teneur en glucose, SIGMA-ALDRICH^{®}) supplémenté avec 0,05 mg/mL de gentamycine, 2 UI/mL d'héparine, 0.001% de 2-Mercaptoethanol (SIGMA-ALDRICH^{®}), 1% d'acide aminé non essentiel, et 10% de lysat plaquettaire.

Le lysat plaquettaire a été préparé à partir de plasma PRP *(Plaquette Rich Plasma)* obtenu auprès de la banque du sang française (Etablissement Français du Sang) et testé négatif pour les virus HIV, HTLV, HCV et HBV. Les sacs contenant le plasma PRP sont stockés à -80°C et réchauffés à 37°C dans un bain-marie. Après réchauffement, le plasma PRP de multiples donneurs sont mélangés et centrifugés à 14 000 G pendant 10 min pour retirer les particules et membranes plaquettaires. Le surnageant du lysat plaquettaire a été collecté et congelé à -80°C jusqu'à utilisation. Le lysat plaquettaire a été testé pour l'absence d'endotoxines bactériennes, l'hémoglobine (pureté), le pH, les protéines totales, l'albumine, l'osmolarité, la stérilité et l'absence de mycoplasme.

Ont été sélectionnées les cellules qui adhérent au plastique de boîte de culture (après 24h de contact avec la surface du plastique), et qui présentent le panel de marqueurs des MSC (cellules positives pour CD105, CD73 et CD90, et négatives pour CD45, CD34, CD14, CD19 et HLA-DR). Ces cellules sont capables de se différencier en adipocytes, chondroblastes et ostéocytes.

Les MSC isolées peuvent être placées en culture sur du milieu d'expansion jusqu'à utilisation (le milieu étant renouvelé lorsque les cellules atteignent la confluence, le décollement des cellules se faisant à la trypsine pendant 5 min à 37°C.

Lors du processus de préparation les MSC peuvent être éventuellement congelées. On peut congeler des MSC jusqu'au moins au 10ème passage. Après décongélation les MSC peuvent être à nouveau amplifiées dans les milieux d'amplification classique avant d'être associées au support. Les biomatériaux obtenus ne seront alors plus congelés pour stockage, délivrance et transfert. Induction de la différenciation des cellules souches mésenchymateuses en cellules formant un tissu contractile contenu dans le support solide poreux.

Les supports solides poreux ont été placés dans des plaques à puits de culture à raison d'un support par puits. Les MSC isolées ont été apportées à raison de 10⁵ cellules par support dans un volume de 10 µL de milieu d'expansion (contenant du lysat plaquettaire, et donc du TGFbêta1). Elles peuvent être déposées à la surface du support solide poreux. Les plaques sont incubées à 37°C pendant 9 à 30 jours. Le milieu d'expansion a été renouvelé tous les deux jours. Les propriétés contractile et immunosuppressive des cellules sont préservées pendant cette période. Après 1 mois les enzymes métalloprotéinases sécrétées par les MSC vont conduire progressivement à une destruction du support et une moindre contractilité si les préparations sont maintenues à 37°C. Les MSC migrent dans le volume du support solide poreux et le colonisent de manière homogène. Au bout de quatre jours, les MSC se sont massivement (à plus de 80%) différenciées en cellules formant un tissu contractile à l'intérieur des pores du support solide poreux à partir du 8eme jour. Si souhaité, l'incubation peut être poursuivie jusqu'à au moins 45 jours.

Le support collagénique que nous avons employé peut être digéré avec des collagénases de type Il (pendant 15 min-1h. à 37°C). Les cellules peuvent alors être récupérées et il est possible d'analyser leur nombre, leur survie, et nous avons développé une cytométrie spécifique pour alphaSMA afin de quantifier ces cellules dans la préparation. Il est possible également d'effectuer un co-marquage pour les cellules alphaSMA et les autres marqueurs mésenchymateux. Cependant dans nos préparations en accord avec notre analyse histologique semi-quantitative, la majorité des cellules sont des cellules alphaSMA et expriment les marqueurs de cellules mésenchymateuses. Dans les supports la viabilité des cellules et de l'ordre de 95% hors condition de stress. Nous n'avons pas mis de différence de survie en présence ou l'absence de RGD. Il n'y a que peu de prolifération cellulaire seulement 20-30% sur 2 semaines. Par contre on observe une différenciation massive des cellules. Entre les matrices avec ou sans RGD, le niveau de différenciation est le même, par contre l'association d'alphaSMA à l'appareil contractile peut être différente et pourrait expliquer la différence de contractilité des différentes préparations.

Nous avons également analysé ces cellules en microscopie classique. Nous n'avons pas observé de différence entre l'extérieur et l'intérieur du support. De manière intéressante initialement les cellules sont capables de coloniser n'importe quel type de support. On peut les mettre à l'extrémité d'un support cylindrique et elles vont coloniser le support probablement du fait de la taille de ses pores. La colonisation se fait plutôt de l'intérieur vers l'extérieur. Si l'on applique sur des préparations jeunes de cellules, les cellules vont passer d'un support à l'autre en formant des ponts. Secondairement il se forme une couche assez importante en surface. Cependant si l'on applique des supports au contact de ces cellules elles ne migrent plus. Nous avons confirmé en microscopie confocale la colocalisation pour les marqueurs alphaSMA et myosine.

Le tissu contractile comprend des cellules qui expriment le marqueur myofibroblastique alphaSMA. Il s'agit donc de cellules contractiles.

En microscopie électronique, même en l'absence de molécules de peptide RGD, on a pu observer des cellules avec une organisation de bandes de stress et une organisation de type sarcomérique, avec une alternance de bandes environ tous les micromètres : *cf.* Figure 1.

Au microscope électronique, les cellules différenciées à partir des MSC ont présenté les caractéristiques de myofibroblastes avec la présence de membrane basale, un réticulum endoplasmique très développé, une organisation particulaire des cellules avec la matrice extracellulaire à type de fibronexus avec et des jonctions intercellulaires de type jonction adhérente. En Figure 1, les rectangles tracés délimitent les zones identifiables de protéines filaments contractiles. Les flèches montrent une interruption régulière de ces filaments par des denses bodies ou peut être un sarcomère. Quelle que soit l'organisation de l'appareil, on observe ici uniquement dans le support de contractilité de la demande, une organisation terminale de l'appareil de contractile.

De manière intéressante, les cellules mésenchymateuses exprimaient également des bandes de stress avec une alternance de bandes de 1 micromètre faisant suspecter une organisation de type sarcomérique. Ces structures sont abondantes et pas seulement au niveau de la zone d'adhérence membranaire. Nous avons vu ces structures dans les supports de collagène avec et sans molécule d'adhésion comme le RGD, et également dans les supports réticulés chimiquement.

Ceci met en évidence *in vitro* le lien formel entre cellules souches mésenchymateuses et cellules contractiles de type myofibroblastiques. Nous montrons que les MSC *in vitro* peuvent se différencier en cellules autres que chondrocytes, ostéocytes et adipocytes, à savoir en myofibroblastes complètement différenciés. Les interactions entre les cellules et la matrice extracellulaire, et entre cellules entre elles, sont extrêmement développées.

Ces structures organisées de l'appareil contractile *in vitro* n'ont à notre connaissance pas été mises en évidence sur des cellules contractiles dérivées de MSC dans des cultures 2D. Plus particulièrement, ces bandes de stress et l'organisation terminale de l'appareil contractile n'ont à notre connaissance jamais été mises en évidence avec des cultures dérivées de MSC. Le biomatériau de la demande permet donc la différenciation terminale des cellules contractiles. Des tests réalisés en l'absence lysat plaquettaire montrent une absence de différenciation des MSC en tissu contractile. L'ajout de TGFbêta1 restaure la différenciation des MSC en tissu contractile dans le support solide poreux.

Il est également possible d'utiliser des MSC présentes dans des préparations de cluster cellulaire comme des préparations de « cardiosphères ». Les cardiosphères sont les cellules actuellement en développement pour la régénération après infarctus. On peut utiliser des cardiosphères allogéniques ou autologues. Des clusters cellulaires humains *(cf.* « cardiosphère ») sont obtenus à partir de biopsies myocardiques humaines et sont enrichis en cellules souches de type MSC et cellules C-kit+.

Les cardiosphères ont été mises *in vitro* dans des matrices de collagène DHT RGD ou des mousses de gélatine réticulées par EDC/NHS pendant 7 jours. Dans le support 3D de collagène RGD les cellules, les cellules migrent en dehors du cluster et colonisent l'intégralité du support. Il existe une augmentation importante des marqueurs cardiaques *(cf.* Figure 5). Il existe également une diminution des protéines du Stress Hsp70, Hsp72 et Hsp90. Dans d'autres préparations cellulaires, nous avons également constaté une activation de la voie de survie Akt dans les cellules associées au support. L'activation de l'Akt pourrait en partie expliquer la meilleure survie des cellules en condition de stress hypothermique.

### Exemple 2 : contractilité du biomatériau

La contractilité des biomatériaux de l'exemple 1 ci-dessus a été testée par stimulation électrique (contraction tétanique provoquée en courant alternatif) et par stimulation chimique au KCI (0.05 M), en suivant le même protocole que celui qui a été appliqué sur du tissu placentaire comme décrit dans Lecarpentier et al. 2013 (Placenta 34 : 1163-1169) et Lecarpentier et al. 2015 (PLoS ONE 10(11) : e0142471).

Les biomatériaux de l'exemple 1 ont été conservés au réfrigérateur à une température supérieure à 0°C et inférieure ou égale à 4°C jusqu'à utilisation. Ils ont ensuite été placés dans un bain à 30°C pendant 30 min, et soumis aux tests de contractilité.

Pour les stimulations mécaniques les tests peuvent être effectués après simple réchauffement à température ambiante. Les préparations sont extrêmement stables durant toutes les mesures qui s'effectuent sur plusieurs heures avec les mêmes réponses en début et fin d'expérience. On peut remettre les préparations au frigo et observer le même type de réponse les jours suivants.

Après stimulation électrique en tétanus, il y a un important raccourcissement des supports. Ce raccourcissement cesse lorsque la stimulation cesse *(cf.* Figure 2). De manière intéressante, la cinétique de la contraction et de la relaxation sont très similaires avec un retour à l'état basal avec la même cinétique. Comme la contraction, la relaxation est un phénomène provoqué par le contingent cellulaire associé au support. La relaxation est un mécanisme dynamique et non passif (si la relaxation était passive, il y aurait une difficulté au remplissage du coeur, qui pourrait conduire à une insuffisance cardiaque diastolique par difficulté de remplissage ventriculaire). De manière remarquable, les cinétiques de contraction et de relaxation sont proches de celles du tissu placentaire.

Les tests ont alors été reproduits en maintenant le stimulus de contraction (courant électrique ou KCI) et en ajoutant dans le milieu un inhibiteur de l'interaction actine-myosine (à savoir le 2,3-butanedione monoxime (BDM) à 4 mM), ou un activateur de la voie de l'oxyde nitrique et du monophosphate de guanosine cyclique (NO-cGMP) (à savoir le dinitrate d'isosorbide (ISD ou RISORDAN^{™}) à 1,7 10⁻⁴ M). L'induction de NO dans les préparations signifie que les préparations sont viables et extrêmement fonctionnelles puisque au moins 4 cascades intracellulaires sont mises en jeu pour obtenir la relaxation.

Les résultats sont illustrés par la Figure 3A (cas d'un biomatériau avec matrice de collagène réticulé par DHT et avec molécules de peptide RGD, sous stimulation électrique en tetanus et avec inhibition par RISORDAN^{™}) et par la Figure 3B (cas d'un biomatériau avec matrice de collagène réticulée par DHT, sous stimulation par KCI 0,05 M, et avec inhibition par BDM). On a pu observer que l'inhibiteur (RISORDAN^{™} ou BDM) induit une relaxation du même si le stimulus ayant provoqué la contraction (courant électrique ou KCI) est maintenu. Ceci démontre que la relaxation est un phénomène actif médié par les unités contractiles (actine myosine) du tissu du biomatériau.

Il a été observé que la présence de molécules d'adhésion (peptide RDG) augmente les paramètres de contractilité, en général d'un facteur de 2 ou 3.

De manière tout à fait exceptionnelle une courbe de Hill a pu être établie *(cf.* Figure 4), qui est caractéristique des tissus contractiles musculaires après stimulation par KCI ou stimulation électrique. Les courbes que nous avons observées sont très superposables à celles qui ont par ailleurs été décrites pour du tissu placentaire humain explanté.

Les complaisances d'élasticité des supports ont été déterminées. Pour le support de collagène la complaisance d'élasticité sans cellules était de 0,4 KPa⁻¹. La complaisance d'élasticité des supports colonisés par les cellules était de 1.5 KPa⁻¹.

### Exemple 3 : induction d'une activité paracrine

Des lymphocytes T ont été marqués avec le marqueur de fluorescence intracellulaire cytoplasmique CFSE (dont la fluorescence est divisée par deux à chaque division cellulaire), et ont été activés par antiCD3 et antiCD28 pendant 48h.

Les biomatériaux de l'exemple 1 ont été incubés avec de l'IFNgamma à 500 UI/mL pendant 7 jours.

Avant et après cette incubation avec l'IFNgamma, les surnagent des biomatériaux ont été placés en contact avec les lymphocytes T activés et marqués.

La consommation en tryptophane a été mesurée dans le milieu d'incubation (selon la méthode Boxam et Warren). La consommation du tryptophane dans le milieu dans les MSC est due à l'activation de l'IDO. Dans le milieu d'incubation des biomatériaux non stimulés par IFNgamma, il n'y a quasiment pas de consommation de trytophane. Par contre, dans le milieu d'incubation des biomatériaux stimulés par IFNgamma, une forte consommation de trytophane a été observée. Elle est du même ordre de grandeur que celle qui serait observée avec une culture standard 2D de cellules MSC non différenciées. Or, il y a très peu de prolifération cellulaire dans le biomatériau par rapport à une culture (standard 2D) de MSC non différenciées. La fonction paracrine du biomatériau est donc supérieure à celle d'une culture (standard 2D) de MSC non différenciées.

La fluorescence CFSE a été mesurée. On peut observer que le biomatériau (avec MSC différenciées en myofibroblastes à plus de 80%) inhibe la prolifération des lymphocytes B qu'il soit à base de collagène réticulé ou de collagène réticulé par DHT avec molécules de peptide RGD.

L'expression de différents facteurs liés à une fonction paracrine, et notamment l'expression de cytokines et de métalloprotéinases, a été mesurée.

Il a été notamment observé (en particulier à J7) une augmentation de l'expression des métalloprotéinases MMP9, MMP2 et MMP14, après stimulation par IFNgamma du biomatériau, et notamment une augmentation de la sécrétion de MMP9 et MMP2, plus particulièrement de MMP9.

Il a donc été observée que, bien que, dans le biomatériau, les MSC se soient différenciées en cellules formant un tissu contractile, l'activité paracrine inductible n'a pas été perdue. Elle apparaît même augmentée.

### Exemple 4 : conservation hors congélation

Les biomatériaux de l'exemple 1 ont été conservés pendant 10 jours dans un réfrigérateur (à une température d'environ 4°C). La contractilité et la fonction paracrine de ces biomatériaux ont été mesurées comme décrit en exemples 2 et 3 ci-dessus. Aucune altération de contractilité ou de fonction paracrine n'a été détectée. Les biomatériaux peuvent donc être conservés hors congélation, et sont immédiatement fonctionnels.

Par contraste, des MSC (non différenciées) conservées par congélation ne reprennent une fonction paracrine qu'au bout de 7 jours après décongélation.

Les biomatériaux ont également pu être envoyés par la poste classiquement dans des containers de polystyrène. Les biomatériaux étant dans leur milieu de culture contenant du sérum et des antibiotiques dans des tubes de 50 mL. À l'intérieur du polystyrène pour maintenir la température basse aux alentours de zéro degré avec des blocs réfrigérants pour glacière « pain de glace » et éventuellement de la carboglace en évitant les contacts directs avec le tube contenant le biomatériau. À leur arrivée les tubes contenant les biomatériaux sont stockés au frigo entre 0-4°C. Dans certains cas des préparations sont arrivées après 6 jours sans problème particulier même si la carboglace n'était plus présente.

Des packs de glaces permettant d'éviter la congélation comme des matériaux utilisés pour délivrer les produits pharmaceutiques de type CRYOPAC permettant de maintenir des températures déterminées de préférence ici entre +2°C et +8°C peuvent être utilisées. On peut éventuellement monitorer la température dans le container. Si on souhaite changer le milieu des préparations, les biomatériaux avec les cellules peuvent être mis dans un nouveau container avec le milieu adéquat. Eventuellement le changement peut être fait au bloc opératoire ou sous une hôte à flux laminaire adapté.

Une autre solution, pour ne pas exposer la préparation à l'air libre est de faire cette modification en milieu confiné.

Une solution peut être d'avoir plusieurs réservoirs en séries avec dans le premier réservoir le milieu final désiré, dans le réservoir du milieu les cellules avec support et le 3ème réservoir pouvant être vide et servir de poubelle. On peut varier sur le nombre de réservoir et leur organisation. Ils n'ont pas tous forcément le même volume. Il peut y avoir présence de communication par des canaux entre les réservoirs avec la possibilité d'avoir des systèmes de fermeture ou antiretour. Il peut également y avoir des systèmes de filtre bactérien.

### Exemple 5 : thérapie post-infarctus myocardique sur modèle animal

Les biomatériaux de l'exemple 1 ont été administrés à des rats servant de modèles d'infarctus du myocarde.

Un modèle d'infarctus myocardique a été développé chez le rat comme décrit dans Vallée et al. 2012 (Stem Cells Translational Medicine 1 :248-260). Des rats OFA (Oncins France souche A), de 250 g chacun, ont été obtenus auprès de la société CHARLES RIVER (Wilmington MA ; U.S.A.). Les rats ont été immunodéprimés par tacrolimus (8 mg/kg/j. en deux injections) avant l'opération et pendant 45 j. Les rats ont été anesthésiés par un mélange de kétamine 100 mg/kg et xylazine 10 mg/kg administré par injection intra-péritonéale, et ont été ventilés après intubation trachéale. Une thoracotomie gauche a été pratiquée pour aborder le coeur et la cavité péricardique. L'infarctus myocardique a été produit par une ligature définitive de l'artère intraventriculaire antérieure au fil de suture PROLENE^{™} 6/0. L'infarctus s'accompagne d'une élévation du segment ST sur l'électrocardiogramme, d'une cyanose localisée sur le territoire en aval du vaisseau occlus et d'une akinésie de la zone (en l'occurrence du myocarde antérieur).

La taille de l'infarctus et les fonctions du ventricule gauche ont été mesurées par IRM à J2, J7 et J45. La ligature de l'artère intraventriculaire antérieure provoque un infarctus de 25% en taille, qui reste stable dans le temps. La fraction d'éjection du ventricule gauche (qui est de 68% à la normale hors ligature et infarctus) passe à 53% à J2, puis à 45% à J7 et J45.

Les biomatériaux de l'exemple 1 ont été conservés pendant 7 jours au réfrigérateur (température de 4°C), puis ont été placés en contact avec de l'IFNgamma pendant 5 jours à température ambiante. Les biomatériaux ont été placés sur la zone infarcie (avec suture optionnelle au fil PROLENE^{™}).

La taille de l'infarctus n'est plus que de 10% à J7 (biomatériau de l'exemple 1 avec matrice de collagène réticulé par DHT et MSC différenciées en myofibroblates), alors qu'elle reste à 25% avec une injection de MSC non différenciées.

La fraction d'éjection du ventricule gauche est de 60% à J2, 59% à J7 et 58% à J45 biomatériau de l'exemple 1 avec matrice de collagène réticulé par DHT et MSC différenciées en myofibroblates, alors qu'elle est de 54%, 52% et 49% à J2, J7 et J45, respectivement, avec un biomatériau dans lequel la différenciation des MSC n'a pas été induite (biomatériau non contractile).

Le biomatériau de l'exemple 1 (matrice de collagène réticulé par DHT ; MSC différenciées en myofibroblates) permet de réduire la dilation ventriculaire après l'infarctus notamment la distension diastolique du ventricule. Il permet en outre d'augmenter la contractilité segmentaire systolique, ce à quoi ne parvient pas le biomatériau non contractile (différenciation des MSC non induite).

Les cellules MSC humaines transfectées avec la RLuc ont été identifiées *in vivo* après injection du substrat pour la luciférase à J1, J4, J8 et J25. Avec les patches de collagènes avec des cellules multipotentes non contractiles il y a une baisse massive à J4 de l'ordre de 75% alors que cette baisse est retardée avec les patches contractiles contenant ou pas du RGD. À 1 mois avec les patches contractiles à base de collagène, 30% des cellules sont encore présentes et 50% si les patches contractiles contiennent le collagène-RGD.

### Exemple 6 : possibilité d'utiliser cette approche en dehors du coeur

*In vivo* le support de collagène améliore la rétention initiale des cellules au contact de tissu musculaire des cellules multipotentes indifférenciées. Les cellules MSC ont cependant dans les matrices de collagène une migration secondaire. La différenciation des MSC en cellules contractiles permet de retarder cette migration à plus de 2 semaines. Il a été montré que les MSC ne doivent pas être forcément présentes de manière permanente mais leur niveau d'initial de prise de greffe détermine une grande partie de leur effet.

Nous avons développé un système de traçage des cellules *in vivo* après transfection de ces cellules avec le gène de la luciférase. Les MSC humaines multipotentes ont été marquées. Les cellules MSC multipotentes ont été cultivées pendant 48h dans les supports. Après 4 jours seulement 50% des cellules localement ont disparu et il reste moins de 10% des cellules à partir du 8ème jour jusqu'à 1 mois. Ces résultats sont cependant supérieurs à ceux obtenus si les MSC sont injectées en libre dans la cuisse où moins de 10% des cellules sont présentes après 24h.

Donc la présence des cellules dans un support améliore leur rétention à J1. Nous avons comparé des supports avec des cellules différentiées J9 en structures contractiles. Avec ces patches à J4 pratiquement 100% des cellules sont encore présentes. À partir de J8 il y a une baisse du nombre de cellules et 50% des cellules à J11. À 1 mois il reste encore 20% des cellules. Avec une légère supériorité des patches collagéniques avec le RGD. La différenciation des MSC dans des structures contractiles diminue la migration secondaire de ces cellules *in vivo.*

## Revendications

1. Procédé de production d'un biomatériau cellulaire contractile, **caractérisé en ce que** le procédé comprend :
- placer des cellules souches mésenchymateuses à l'intérieur d'un support tridimensionnel, ledit support présentant des pores permettant la diffusion des nutriments, et la colonisation cellulaire in vitro, et in vivo,
- induire la différenciation des cellules souches mésenchymateuses multipotentes en cellules contractiles, choisies parmi les myofibroblastes, les myocytes, les cardiomyocytes, ou les cellules musculaires striées, dans un support tridimensionnel à complaisance d'élasticité qui permet la formation d'un tissu contractile par la mise en contact desdites cellules souches mésenchymateuses avec un agent favorisant la différenciation choisi parmi TGFbêta, CTGF, EGF, NUX1 et le lysat plaquettaire, et
- induire une activité paracrine desdites cellules contractiles, l'activité paracrine induite comprenant de plus une activité d'immunosuppression de cellules T et/ou activation de l'indoleamine 2,3-dioxygenase (IDO), ladite activité paracrine est induite par la mise en contact de ces cellules contractiles avec au moins un agent inducteur d'activité paracrine choisi parmi IFNgamma, TNFalpha et IL1bêta, et/ou l'application d'une hypoxie transitoire sur ces cellules contractiles,
dans lequel
ledit support tridimensionnel présente une complaisance d'élasticité sans cellule de 0,1 à 30kPa⁻¹, plus particulièrement de 0.4 à 20 kPa⁻¹, plus particulièrement de 0.5 à 15 kPa⁻¹, plus particulièrement de 0.5 à 5 kPa⁻¹,
ledit support contenant les cellules contractiles ainsi produit étant un biomatériau cellulaire contractile adapté à la thérapie régénérative du tissu myocardique.

2. Le procédé selon la revendication 1, **caractérisé en ce que** ledit support est choisi parmi un polymère naturel choisi parmi le collagène, la gélatine, le fibrinogène, la fibrine, la soie FIBROIN, l'alginate, un protéoglycane, une glycoprotéine, un glycoaminoglycane, un alginate, l'agarose, l'acide hyaluronique, l'agar, le chitosane, le fibrinogène/fibrine, le chitosan carboxyméthylé, l'octasulfate de sucrose, le dextrane, la cellulose, la cellulose méthylée, la sépharose, ou leur association, ou
un polymère synthétique organique choisi parmi le poly (acide lactique) (PGA) et/ou poly (DL-lactide-Co-glycolide) (PLGA) et/ou poly (DL-lactide-Co-caprolactone) (PCL), des polymères synthétiques liés au collagène choisis parmi le Poly(3-hydroxybutyrate) (PHB)+++, la poly(e-caprolactone) (PCL), l'acide poly-lactique (PLA), la polyamide (PA), l'acide polylactique (PLA), l'acide polyglycolique (PGA), le poly (L-lactique) (PLLA), le PLGA, les poly (anhydrides) (PA), le polycarbonate (PC), les hydroxy acides, les poly ortho- esters (POE), les propylfumarates (PPF), les polysaccharides, la polylactone (PL), les poly caprolactones, polyamides, les acides de polyamino, les polyacétals, les polyphosphazènes (PPZ), polycyanoacrylates biodégradables, les polyuréthanes biodégradables (unité centrale), la polypyrrole, les polyanilines, le polythiophène, le polystyrène, le polyester (PE), les polyuréthanes non-biodégradables, les polyurées, le poly (éthylène-téréphtalate) (PET), le poly (acétate de vinyle d'éthylène), le polypropylène, le polyméthacrylate, le polyéthylène, les polycarbonates, le poly oxyde d'éthylène, le poly alcool de vinyle (PVA), le fuseau-tex (polytétrafluoroéthylène), le dacron (téréphtalate de polyéthylène), le polytétrafluoroéthylène (PTFE), le poly-éthylène-glycol (PEG), et le polyglycérol sébacate (PGS).

3. Le procédé selon la revendication 1, **caractérisé en ce que** ledit support est un échafaudage tridimensionnel de polymère réticulé, de préférence de collagène réticulé, qui présente une porosité de 20 à 200 µm, et qui optionnellement comprend des molécules de peptide d'adhésion, de préférence des molécules de peptide RGD, liées de manière covalente à la structure polymérique du support solide.

4. Le procédé selon la revendication 1, **caractérisé en ce que** ledit support est constitué de collagène réticulé, qui présente une porosité de 20 à 200 µm.

5. Le procédé selon la revendication 3 ou 4, **caractérisé en ce que** ledit support comprend des molécules de peptide d'adhésion, plus particulièrement des molécules de peptide RGD, liées de manière covalente à la structure polymérique du support solide.

6. Le procédé selon l'une quelconque des revendications 1 à 5, **caractérisé en ce que** ledit biomatériau est conservé hors congélation, à une température supérieure ou égale à 0°C, à une température supérieure à 0°C, à une température supérieure à 0°C et inférieure à 32°C, à une température supérieure à 0°C et inférieure à 10°C, à une température supérieure à 0°C et inférieure à 4°C ;
et est immédiatement disponible et fonctionnel.

## Patentansprüche

1. Verfahren zur Herstellung eines kontraktilen zellulären Biomaterials, **dadurch gekennzeichnet, dass** das Verfahren Folgendes umfasst:
- Platzieren mesenchymaler Stammzellen im Inneren eines dreidimensionalen Trägers, wobei der Träger Poren aufweist, die die Diffusion von Nährstoffen und die Zellbesiedelung in vitro und in vivo ermöglichen,
- Induzieren der Differenzierung multipotenter mesenchymaler Stammzellen in kontraktile Zellen, ausgewählt aus Myofibroblasten, Myozyten, Kardiomyozyten oder quergestreiften Muskelzellen, in einem dreidimensionalen Träger mit Elastizitätsnachgiebigkeit, der die Bildung eines kontraktilen Gewebes durch in Kontakt bringen der mesenchymalen Stammzellen mit einem die Differenzierung fördernden Mittel gestattet, ausgewählt aus TGFbeta, CTGF, EGF, NUX1 und Thrombozytenlysat, und
- Induzieren einer parakrinen Aktivität der kontraktilen Zellen, wobei die induzierte parakrine Aktivität außerdem eine Immunsuppressionsaktivität von T-Zellen und/oder eine Aktivierung von Indolamin-2,3-Dioxygenase (IDO) umfasst, wobei die parakrine Aktivität durch in Kontakt bringen der kontraktilen Zellen mit mindestens einem die parakrine Aktivität induzierenden Wirkstoff induziert wird, ausgewählt aus IFNgamma, TNFalpha und IL1beta, und/oder die Anwendung einer vorübergehenden Hypoxie auf die kontraktilen Zellen,
wobei
der dreidimensionalen Träger eine Elastizitätsnachgiebigkeit ohne Zelle von 0,1 bis 30 kPa⁻¹, insbesondere von 0,4 bis 20 kPa⁻¹, insbesondere von 0,5 bis 15 kPa⁻¹, insbesondere von 0,5 bis 5 kPa⁻¹ aufweist,
wobei der Träger, der die so hergestellten kontraktilen Zellen enthält, ein kontraktiles zelluläres Biomaterial ist, das für die regenerative Therapie von Myokardgewebe angepasst ist.

2. Verfahren nach Anspruch 1, **dadurch gekennzeichnet, dass** der Träger ausgewählt wird aus einem natürlichen Polymer, ausgewählt aus Kollagen, Gelatine, Fibrinogen, Fibrin, FIBROIN-Seide, Alginat, einem Proteoglykan, einem Glykoprotein, einem Glykoaminoglykan, einem Alginat, Agarose, Hyaluronsäure, Agar, Chitosan, Fibrinogen/Fibrin, Carboxymethylchitosan, Saccharoseoctasulfat, Dextran, Cellulose, methylierter Cellulose, Sepharose oder deren Kombination, oder
einem synthetischen organischen Polymer, ausgewählt aus Poly(milchsäure) (PGA) und/oder Poly(DL-lactid-Co-glycolid) (PLGA) und/oder Poly(DL-lactid-Co-caprolacton) (PCL), mit Kollagen verbundenen synthetischen Polymeren, ausgewählt aus Poly(3-hydroxybutyrat) (PHB)+++, Poly(e-caprolacton) (PCL), Polymilchsäure (PLA), Polyamid (PA), Polymilchsäure (PLA), Polyglykolsäure (PGA), Poly(L-Milchsäure) (PLLA), PLGA, Poly(anhydriden) (PA), Polycarbonat (PC), Hydroxysäuren, Polyorthoestern (POE), Propylfumaraten (PPF), Polysacchariden, Polylacton (PL), Polycaprolactonen, Polyamiden, Polyaminosäuren, Polyacetalen, Polyphosphazenen (PPZ), biologisch abbaubaren Polycyanoacrylaten, biologisch abbaubaren Polyurethanen (Zentraleinheit), Polypyrrol, Polyanilinen, Polythiophen, Polystyrol, Polyester (PE), nicht biologisch abbaubaren Polyurethanen, Polyharnstoffen, Poly(ethylenterephthalat) (PET), Poly(Ethylenvinylacetat), Polypropylen, Polymethacrylat, Polyethylen, Polycarbonaten, Polyethylenoxid, Polyvinylalkohol (PVA), Fuseau-tex (Polytetrafluorethylen), Dacron (Polyethylenterephthalat), Polytetrafluorethylen (PTFE), Polyethylenglykol (PEG) und Polyglycerolsebacat (PGS).

3. Verfahren nach Anspruch 1, **dadurch gekennzeichnet, dass** der Träger ein dreidimensionales Gerüst aus vernetztem Polymer, vorzugsweise vernetztem Kollagen ist, das eine Porosität von 20 bis 200 µm aufweist und wahlweise Adhäsionspeptidmoleküle umfasst, vorzugsweise RGD-Peptidmoleküle, die kovalent an die Polymerstruktur des festen Trägers gebunden sind.

4. Verfahren nach Anspruch 1, **dadurch gekennzeichnet, dass** der Träger aus vernetztem Kollagen besteht, das eine Porosität von 20 bis 200 µm aufweist.

5. Verfahren nach Anspruch 3 oder 4, **dadurch gekennzeichnet, dass** der Träger Adhäsionspeptidmoleküle, insbesondere RGD-Peptidmoleküle umfasst, die kovalent an die Polymerstruktur des festen Trägers gebunden sind.

6. Verfahren nach einem der Ansprüche 1 bis 5, **dadurch gekennzeichnet, dass** das Biomaterial ohne Gefrieren bei einer Temperatur größer oder gleich 0 °C, bei einer Temperatur größer als 0 °C, bei einer Temperatur größer als 0 °C und unter 32 °C, bei einer Temperatur über 0 °C und unter 10 °C, bei einer Temperatur über 0 °C und unter 4 °C gelagert wird;
und sofort verfügbar und funktionsfähig ist.

## Claims

1. A method for producing a contractile cellular biomaterial, **characterized in that** the process comprises:
- placing mesenchymal stem cells inside a three-dimensional support, said support having pores allowing the diffusion of nutrients, and cell colonization in vitro, and in vivo,
- inducing the differentiation of multipotent mesenchymal stem cells into contractile cells, chosen from myofibroblasts, myocytes, cardiomyocytes, or striated muscle cells, in a three-dimensional support with elasticity complacency that allows the formation of contractile tissue by bringing said mesenchymal stem cells into contact with a differentiation-promoting agent chosen from TGFbeta, CTGF, EGF, NUX1 and platelet lysate, and
- inducing paracrine activity of said contractile cells, the induced paracrine activity also including T cell immunosuppression activity and/or indoleamine 2,3-dioxygenase (IDO) activation, said paracrine activity is induced by the contact of these contractile cells with at least one paracrine activity inducing agent chosen from IFNgamma, TNFalpha and IL1beta, and/or the application of transient hypoxia on these contractile cells,
in which
said three-dimensional support has a compliance without a cell from 0.1 to 30kPa⁻¹, especially from 0.4 to 20 kPa⁻¹, especially from 0.5 to 15 kPa⁻¹, especially from 0.5 to 5 kPa⁻¹,
said carrier containing the contractile cells thus produced being a contractile cell biomaterial suitable for the regenerative therapy of myocardial tissue.

2. The method according to claim 1, **characterized in that** said support is chosen from a natural polymer selected from collagen, gelatin, fibrinogen, fibrin, FIBROIN silk, alginate, a proteoglycan, a glycoprotein, a glycoaminoglycan, an alginate, agarose, hyaluronic acid, agar, chitosan, fibrinogen/fibrin, carboxymethylated chitosan, sucrose octasulfate, dextran, cellulose, methylated cellulose, sepharose, or a combination thereof, or
an organic synthetic polymer selected from poly(lactic acid) (PGA) and/or poly (DL-lactide-Co-glycolide) (PLGA) and/or poly (DL-lactide-Co-caprolactone) (PCL), collagen-bonded synthetic polymers selected from Poly(3-hydroxybutyrate) (PHB)+++, poly(e-caprolactone) (PCL), poly-lactic acid (PLA), polyamide (PA), polylactic acid (PLA), polyglycolic acid (PGA), poly(L-lactic) (PLLA), PLGA, poly (anhydrides) (PA), polycarbonate (PC), hydroxy acids, polyortho-esters (POE), propyl fumarates (PPF), polysaccharides, polylactone (PL), polycaprolactones, polyamides, polyamino acids, polyacetals, polyphosphazen (PPZ), biodegradable polycyanoacrylates, biodegradable polyurethanes (PU), polypyrrole, polyanilines, polythiophene, polystyrene, polyester (PE), non-biodegradable polyurethanes, polyureas, polyethylene terephthalate (PET), poly (ethylene vinyl acetate), polypropylene, polymethacrylate, polyethylene, polycarbonates, polyethylene oxide, polyvinyl alcohol (PVA), spindle-tex (polytetrafluoroethylene), dacron (polyethylene terephthalate), polytetrafluoroethylene (PTFE), polyethylene glycol (PEG), and polyglycerol sebacate (PGS).

3. The method according to claim 1, **characterized in that** said support is a three-dimensional cross-linked polymer scaffold, preferably cross-linked collagen, which has a porosity of 20 to 200 µm, and which optionally comprises adhesion peptide molecules, preferably RGD peptide molecules, covalently bonded to the polymeric structure of the solid support.

4. The method according to claim 1, **characterised in that** said support is made of cross-linked collagen, which has a porosity of 20 to 200 µm.

5. The method according to claim 3 or 4, **characterized in that** said carrier comprises adhesion peptide molecules, in particular RGD peptide molecules, covalently bonded to the polymeric structure of the solid support.

6. The method according to any one of claims 1 to 5, **characterised in that** said biomaterial is kept out of freezing, at a temperature greater than or equal to 0°C, at a temperature greater than 0°C, at a temperature greater than 0°C and below 32°C, at a temperature greater than 0°C and below 10°C, at a temperature above 0°C and below 4°C;
and is immediately available and functional.
